# EUROPÄISCHE PATENTSCHRIFT

(11) **EP 4 243 730 B1**
(45) Veröffentlichungstag und Bekanntmachung des Hinweises auf die Patenterteilung: **04.11.2026**
(21) Anmeldenummer: 21810961.9
(22) Anmeldetag: 10.11.2021
(51) Int. Cl.: A61F 2/16, A61B 90/94, A61F 9/00, G02C 7/02, G02C 7/04, B29D 11/00, B29D 11/02

(54) **OPHTHALMOLOGISCHES IMPLANTAT MIT DIGITALER PRODUKTKENNZEICHNUNG**
OPHTHALMOLOGICAL IMPLANT WITH DIGITAL PRODUCT IDENTIFIER
IMPLANT OPHTALMOLOGIQUE POURVU D'UNE IDENTIFICATION PRODUIT NUMÉRIQUE

(30) Priorität: 10.11.2020 DE 102020214126; 15.06.2021 DE 102021206092
(43) Veröffentlichungstag der Anmeldung: 20.09.2023
(73) Patentinhaber: Carl Zeiss Meditec AG, 07745 Jena (DE)
(72) Erfinder: GERLACH, Mario, 10589 Berlin (DE); SCHREIBER, Benjamin, 10589 Berlin (DE); MASCH, Jennifer-Magdalena, 23909 Ratzeburg (DE); BADUR, Thorben, 10627 Berlin (DE); WOLFSTEIN, Andre, 13507 Berlin (DE)
(74) Vertreter: Rößner, Ulrike
(86) Internationale Anmeldenummer: PCT/EP2021/081210
(87) Internationale Veröffentlichungsnummer: WO 2022/101252

(56) Entgegenhaltungen:
- WO-A2-2005/015290
- DE-A1- 10 159 521
- US-A1- 2011 264 209
- US-A1- 2014 168 600
- US-A1- 2016 144 579
- US-A1- 2018 031 863
- US-A1- 2020 108 466

## Beschreibung

Die vorliegende Erfindung betrifft ein ophthalmologisches Implantat mit einem optisch abbildenden Element und bevorzugt mit einer Haptik, die sich dem optisch abbildenden Element anschließt, wobei auf dem optisch abbildenden Element eine digitale Produktkennzeichnung angeordnet ist. Die vorliegende Erfindung betrifft weiterhin ein entsprechendes Verfahren zu dessen Herstellung sowie ein Maschinenlesesystem zur Erfassung und Decodierung der digitalen Produktkennzeichnung.

Ophthalmologische Implantate, insbesondere kommerzielle Intraokularlinsen (IOL), werden in der Regel mittels Label auf der Primär- und Sekundärverpackung gekennzeichnet. Auf dem Label findet man, neben anderen Herstellerinformationen, den Typ der IOL sowie den Brechwert. Die korrekte Versorgung des Patienten setzt somit voraus, dass die verpackte und ausgelieferte Linse in ihren Eigenschaften den Angaben auf dem Label entspricht. Der Anwender muss sich hier auf die Hersteller verlassen, da eine eindeutige Identifikation bzw. eine Überprüfung im OP nur anhand der visuellen Linsenmerkmale schwierig sind. Diese Art der Kennzeichnung birgt die Gefahr einer Verwechslung, falls ein ophthalmologisches Implantat, insbesondere eine Intraokularlinse der falschen Verpackung zugeführt wird. Dies kann in der Konsequenz unter Umständen zu Explantationen und Produktrückrufen führen.

Intraokularlinsen und andere ophthalmologische Implantate sind Medizinprodukte, bei denen die Rückverfolgbarkeit eine zentrale Anforderung ist. Bei den derzeitigen Produkten enthält die Verpackung der Linse bzw. des Implantats einen Unique Device Identifier (UDI) in Form eines Strichcodes, eines Data-Matrix-Codes oder eines über Funk lesbaren Mikrochips. Ist das ophthalmische Implantat dann in das Auge verbracht worden, so ist sie nicht mehr entsprechend identifizierbar.

Es wurden deshalb Verfahren und Vorrichtungen vorgeschlagen, die ophthalmologischen Implantate individuell zu kennzeichnen, wie zum Beispiel in US 2006/0001828 A1, in der - außerhalb einer optisch wirksamen Zone - ophthalmologische Linsen wie Kontaktlinsen oder Intraokularlinsen mittels eines Matrixcodes gekennzeichnet werden, oder auch andere lasergravierte Matrixcodes in der linsennahen oder aber entfernteren Haptik. Eine Kennzeichnung außerhalb der optisch wirksamen Zone ist bei ophthalmologischen Implantaten jedoch schwer einsehbar, wenn diese erst einmal implantiert sind. Zudem setzt eine Markierung der Haptik mittels lasergraviertem Matrixcode ein entsprechendes Lasersystem voraus.

Die US 2016/0144579 A1 schlägt eine Form zur Fertigung von Kontaktlinsen im Spritzgussverfahren vor, mittels der die Kontaktlinsen schon während der Fertigung mittels einer permanenten Markierung gekennzeichnet werden können, wobei sich diese in einem Randbereich der Kontaktlinse, außerhalb der zentralen optischen Zone befindet. Dies mag für Kontaktlinsen noch eine Lösung sein, aber dieser geometrische Bereich des Codes von ophthalmologischen Implantaten, insbesondere von Intraokularlinsen nach dem Stand der Technik, ist im implantierten Zustand im Auge visuell nicht zugänglich: Die Iris, selbst im medizinisch erweiterten Zustand, versperrt die Sicht auf den haptischen Bereich. Hier ist es erwünscht, den Code z.B. in der Biometrie oder über ein Mikroskop (z.B.: Spaltlampe oder Operationsmikroskop) zugänglich zu machen. Nach der Norm ISO 11979-2, die die derzeitigen Anforderungen an ophthalmologische Linsen beschreibt, sind Markierungen auf dem optisch abbildenden Element der IOL zulässig, wenn eine klare Zone von 4,4 mm eingehalten wird. So wird z. B. häufig eine torische Markierung außerhalb des 4,4-mm-Bereichs verwendet, um torische IOLs im Auge auszurichten, und diese Markierungen sind visuell mit einigen Mühen zugänglich.

Die WO 2009/124838 A2 hingegen beschreibt ein ophthalmologisches Implantat mit einer Markierung, die auch auf einem optisch abbildenden Element möglich ist. Allerdings wird zur Markierung in diesem Fall entweder ein fluoreszierender Farbstoff mit einem Emissionsmaximum außerhalb des für den Menschen sichtbaren Lichtspektrums oder ein absorbierender Farbstoff mit einem Absorptionsmaximum außerhalb des für den Menschen sichtbaren Lichtspektrums verwendet. Das hier verwendete Verfahren ist jedoch technisch sehr aufwändig und erfordert einen zusätzlichen biokompatiblen Fluoreszenzfarbstoff oder absorbierenden Farbstoff und ein komplexes Fluoreszenzanregungs- bzw. Detektionssystem.

Für entsprechend markierte Kontaktlinsen schlägt die WO 2005/015290 A2 eine Möglichkeit des Auslesens von Markierungen mittels eines Bildgebungsverfahrens und einer entsprechenden Beleuchtung, ggf. in Kombination mit einer Wellenfront-Messung zur physikalischen Bestimmung der Linseneigenschaften, vor. Das entsprechende Verfahren ist dadurch sehr komplex und fehleranfällig.

Um fehlerhaftes Auslesen von Markierungen aufzuspüren, schlagen die US 2014/0168600 A1 und die US2011/0264209 A1 den Einsatz von Prüfsummen bei Markierungen auf Kontaklinsen bzw. Intraokularlinsen vor.

Beide hier beschriebenen, aber nicht beanspruchten, Verfahren müssen zudem derart in die Fertigungskette eingebunden werden, dass keine falsche Kennzeichnung der IOLs durch Bedien- oder Programmierfehler möglich ist: Beide erfordern zusätzliche Fertigungsschritte unter Nutzung zusätzlicher Werkzeuge, um die Markierungen zu realisieren.

Aufgabe der vorliegenden Erfindung ist es deshalb, ein ophthalmologisches Implantat, insbesondere eine Intraokularlinse, und ein Verfahren zu dessen Herstellung zu beschreiben, das eine eindeutige und vollständige Produktkennzeichnung (UDI), z.B. Typ, Brechwert, Seriennummer, Chargennummer auf der Intraokularlinse, und Überprüfung dieser mit einfachen Mitteln und zu jedem Zeitpunkt zulässt.

Die Erfindung ist in den unabhängigen Ansprüchen definiert. Die abhängigen Ansprüche betreffen bevorzugte Weiterbildungen.

Ein ophthalmologisches Implantat, insbesondere eine Intraokularlinse, umfasst ein optisch abbildendes Element, insbesondere eine zentrale optische Linse, mit einer optisch wirksamen Zone und bevorzugt mit einer Haptik, die sich dem optisch abbildenden Element anschließt. Ophthalmologische Implantate umfassen üblicherweise eine Haptik zur entsprechenden Fixierung dieser Implantate in einem Patientenauge. In besonderen Fällen ist ein ophthalmologisches Implantat jedoch auch ohne Haptik vollständig und implantierbar.

Auf dem optisch abbildenden Element des ophthalmologischen Implantats, bevorzugt innerhalb der optisch wirksamen Zone, ist eine digitale Produktkennzeichnung des ophthalmologischen Implantats, insbesondere beispielsweise des Typs und der Brechkraft und/oder eines Datenbankschlüssels (also eines eindeutigen Identifiers) angeordnet.

Im Falle, dass die digitale Produktkennzeichnung einen Datenbankschlüssel im Sinne eines eindeutigen Identifiers umfasst, sind vorher in einem Datenbanksystem diesem Datenbankschlüssel die implantatsspezifischen Produktinformationen zugeordnet worden. Durch das Auslesen des Datenbankschlüssels auf dem ophthalmologischen Implantat (z.B. durch das im Folgenden beschriebene Maschinenlesesystem, dass dann bevorzugt direkt mit dem Datenbanksystem verbunden ist) kann eine Datenbankabfrage der implantatspezifischen Produktinformationen durchgeführt werden. Mittels dieser Vorgehenswese können bei kleinen Kodierungslängen umfangreiche und eindeutige Produktinformationen zugänglich gemacht werden.

Wird ein Datenbankschlüssel zur eindeutigen Identifikation als digitale Produktkennzeichnung auf dem ophthalmologischen Implantat angeordnet, so wird in der Regel nur dieser Datenbankschlüssel auf dem ophthalmologischen Implantat "hinterlegt" - denn der Datenbankschlüssel gibt schließlich Zugang zu eindeutigen Informationen über das spezifische ophthalmologische Implantat, die in dem Datenbanksystem selbst sehr detailliert hinterlegbar bzw. hinterlegt sind.

Das Datenbanksystem wird vorzugsweise in einem Datennetzwerk oder einer Datencloud zur Verfügung gestellt. In diesem Datenbanksystem hinterlegt beispielsweise der Hersteller zu jedem Datenbankschlüssel, also zu jedem eindeutigen Identifier, die implantatspezifischen Produktinformationen.

Es ist jedoch auch denkbar, dass neben diesem Datenbankschlüssel trotzdem noch die wichtigsten Produktinformationen, also beispielsweise Typ und Brechkraft, als digitale Produktkennzeichnung angeordnet sind, so dass beispielsweise ein behandelnder Arzt an diese wichtigsten Produktkennzeichnungen auch ohne Verbindung zum Datenbanksystem herankommt.

Erfindungsgemäß ist diese digitale Produktkennzeichnung mittels eines im Bereich des sichtbaren Lichts maschinenlesbaren "codierten Markers", hier in Form eines codierten Punktrasters aus Markierungspunkten realisiert, das einen pseudozufälligen unregelmäßigen Charakter hat. "Pseudozufällig unregelmäßig" heißt dabei, dass eine Anzahl verschiedener definierter Abweichungen der Markierungspunkte von einem festen Bezugspunkt, der einen regelmäßigen Charakter des Punktrasters erzeugen würde, besteht. Dadurch können Gitterbeugungseffekte minimieren werden, die bei einem regelmäßigen Punktraster bestehen würden und zu einer Beeinträchtigung der Abbildung mittels eines solchen ophthalmologischen Implantats, und damit beispielsweise zu einer Beeinträchtigung des Sehens mit einer entsprechenden Intraokularlinse, führen würden.

Dabei ist die optisch wirksame Zone des optisch abbildenden Elements, in der die digitale Produktkennzeichnung bevorzugt angeordnet ist, eine Zone, die durch die Pupillenöffnung bei normalen Lichtverhältnissen bestimmt wird.

Es sind zunächst zwei Bereiche für die Markierung des optisch abbildenden Elements, insbesondere der der optisch wirksamen Zone zu unterscheiden:
Die zentrale optische Zone ist der zentrale Bereich mit einem Durchmesser von 4,4 mm; In diesem Bereich ist eine Markierung nach dem derzeitigen Stand nicht ISOkonform.

Die periphere optische Zone ist der äußere Bereich des optisch abbildenden Elements, in dem die zentrale optische Zone mit einem Durchmesser von 4,4 mm frei bleibt; In diesem Bereich ist eine Markierung nach dem derzeitigen Stand ISOkonform. Die optisch wirksame Zone des optisch abbildenden Elements ist damit Teil der zentralen optischen Zone, in der eine Markierung bislang nicht vorgenommen wurde / werden durfte: Dies war nämlich mit Markierungen nach dem Stand der Technik bislang nicht möglich, ohne die visuelle Qualität für den das Implantat (die IOL) tragenden Patienten erheblich zu beeinträchtigen.

Markierungspunkte des maschinenlesbaren codierten Punktrasters sind dabei keine Punkte im mathematischen Sinne, sondern besitzen eine Größe: In der Regel sind sie rund und durch einen Durchmesser gekennzeichnet. In einer Ausführungsform können sie jedoch eine elliptische Form annehmen - weisen also in der Draufsicht eine von der Breite signifikant unterschiedliche Länge auf. Auch möglich sind noch andere geometrische Formen wie Quadrate und Rechtecke.

In dieser Erfindung werden also optische Codes, z. B. Daten-Matrix-Codes, auf oder in dem optisch abbildenden Element einer ophthalmologischen Linse beschrieben. Die hier ausgeführten Systeme und Verfahren basieren auf einer IOL, die mit einem codierten Marker ausgestattet ist. Die Erfindung schließt jedoch andere ophthalmologische Implantate nicht aus, die im Auge positioniert werden müssen, z. B. Kapselspannringe, Stents und ICLs.

Die Ausstattung des Implantats mit einem codierten Marker, auch "Tag" genannt, als Identifikator bietet verschiedene Vorteile. Sie unterstützt die chirurgischen Arbeitsabläufe. Eine erlaubt aber auch die Rückverfolgbarkeit des Implantats von der Produktion, über die Logistik, die Implantation, in-vivo und ggf. nach der Explantation für das Beschwerdemanagement.

Hier steht also u.a. die Anbindung an Produktions- und Operationsgeräte und an die Diagnostik im Fokus. Damit dies möglich ist, wird der codierte Marker auch im implantierten Zustand optisch zugänglich gemacht.

**In** einer bevorzugten Ausführungsform des erfindungsgemäßen ophthalmologischen Implantats ist das maschinenlesbare codierte Punktraster aus Markierungspunkten zentral innerhalb der optisch wirksamen Zone des optisch abbildenden Elements angeordnet. Dadurch ist es auch im implantierten Zustand jederzeit optisch zugänglich und durch eine entsprechendes Maschinenlesesystem sichtbar und auslesbar.

Auch wenn eine zentrale Lage des codierten Punktrasters aus Markierungspunkten innerhalb der optisch wirksamen Zone des optisch abbildenden Elements besonders vorteilhaft für die Zugänglichkeit ist, kann sich das codierte Punktraster aus Markierungspunkten jedoch an beliebiger Position innerhalb der optisch wirksamen Zone oder auch außerhalb der optisch wirksamen Zone befinden.

Die Aufgabe wird also dahingehend gelöst, dass mit dem ophthalmologischen Implantat eine digitale Kennzeichnung in Form eines maschinenlesbaren codierten und im Bereich des sichtbaren Lichts lesbaren Punktrasters verbunden wird, das aber entgegen der üblichen Auslegung des Begriffs "Punktraster" eben keine Regelmäßigkeiten in einer Form aufweist, die zu Gitterbeugungseffekten führen könnten. Deshalb ist dieses Punktraster bevorzugt sogar zentral innerhalb der optisch wirksamen Zone der IOL anordenbar: Es ist durch seine Struktur derart ausgelegt, dass es keine für den Patienten wahrnehmbare negative optische Wirkung hat.

In einer konkreten und besonders vorteilhaften Ausführungsform des erfindungsgemäßen ophthalmologischen Implantats ist das codierte Punktraster aus Markierungspunkten so aufgebaut, dass ein virtuelles polares oder kartesisches Basisgitter auf dem optischen abbildenden Element, bevorzugt auf der optischen Zone des optisch abbildenden Elements, so angeordnet ist, das dadurch gleichartige Sektoren bzw. gleichartige Zellen mit jeweils einem definierten Basisgitterpunkt des Sektors bzw. der Zelle beschrieben werden. Ein solcher (virtueller) Basisgitterpunkt des virtuellen Basisgitters kann als Mittelpunkt des Sektors bzw. der Zelle verstanden werden.

Ein realer Markierungspunkt des Punktrasters ist hingegen in jedem Sektor bzw. in jeder Zelle in einer Position angeordnet, die einen Versatz zu diesem Basisgitterpunkt aufweist, wobei der Versatz in jedem Sektor bzw. jeder Zelle in einer von vier möglichen Richtungen, von denen vorzugsweise je zwei einander entgegengesetzt verlaufen, erfolgt, und einen definierten Abstand zum Basisgitterpunkt aufweist. Je nach Möglichkeiten der Erstellung des codierten Punktrasters aus Markierungspunkten und der Auflösung eines Maschinenlesesystems, das dieses codierte Punktraster wieder auslesen soll, sind natürlich auch mehr als 4 Richtungen des Versatzes möglich. Von daher ist diese besonders vorteilhafte Ausführungsform als "mindestens vier mögliche Richtungen des Versatzes des Markierungspunktes zum Basisgitterpunkt" zu lesen.

In einer Ausführungsform des erfindungsgemäßen ophthalmologischen Implantats wie soeben beschrieben stellt ein Sektor bzw. eine Zelle mit einem Basisgitterpunkt vier Zustände zur Verfügung, die durch die jeweilige Lage des Markierungspunktes in einer von vier Positionen um den Basisgitterpunkt gekennzeichnet sind. Der Markierungspunkt des Sektors oder der Zelle kann also jeweils eine von vier möglichen Positionen um einen Basispunkt eines Sektors bzw. einer Zelle einnehmen, wodurch mit diesem Sektor bzw. mit dieser Zelle vier unterschiedliche Zustände beschrieben werden können.

In einer Weiterentwicklung dieser Ausführungsform des ophthalmologischen Implantats stellt ein Sektor bzw. eine Zelle mit einem Basisgitterpunkt einen weiteren, fünften, Zustand zur Verfügung, der durch das Nichtvorhandensein eines Markierungspunktes an einer der vier möglichen Positionen um einen Basisgitterpunkt definiert ist. Somit ergeben sich für einen Sektor bzw. eine Zelle fünf mögliche Zustände. Das hat den Vorteil, dass die Codierung einer eindeutigen Identifikationsnummer in die fünf möglichen Zustände der Sektoren bzw. Zellen im statistischen Mittel in 1/5 der Fälle zum Nicht-Vorhandensein eines Markierungspunktes in diesem Sektor bzw. in dieser Zelle führt. Somit wird die Anzahl der tatsächlich benötigten Markierungspunkte um ~1/5 reduziert. Dies mindert weiter potentielle optische Auswirkungen des codierten Punktrasters aus Markierungspunkten.

**In** einer weiteren Ausgestaltung des erfindungsgemäßen ophthalmologischen Implantats sind in einem Sektor bzw. in einer Zelle mit einem Basisgitterpunkt weitere Zustände definiert durch weitere mögliche Versatzrichtungen und/oder weitere mögliche definierte Abstände des Versatzes des Markierungspunktes zum Basisgitterpunkt.

Ebenfalls vom Erfindungsgedanken umfasst ist also die zusätzliche Anordnung der Markierungspunkte auf weiteren Positionen innerhalb eines Sektors oder einer Zelle auf den Basiskreuzungspunkten oder weiteren Kreuzungspunkten zwischen Basisgitter und versetztem Gitter. Bei zusätzlicher Nutzung der Basiskreuzungspunkte kann beispielsweise ein Sektor bzw. eine Zelle mit einem (virtuellen) Basisgitterpunkt bis zu neun geometrischen Zuständen erzeugen. Es hängt von der Leistungsfähigkeit des Verfahrens zur Erzeugung der maschinenlesbaren codierten Punktrasters aus Markierungspunkten sowie des Maschinenlesesystems ab, wie viele Positionen eindeutig und sicher erkennbar sind.

Vorteilhaft ist ein erfindungsgemäßes ophthalmologisches Implantat, dessen Anteil der Fläche der Markierungspunkte zur Gesamtfläche des optisch abbildenden Elements kleiner 2%, insbesondere kleiner 1% und besonders bevorzugt kleiner 0,5% ist, und/oder wobei und/oder Anteil der Fläche der Markierungspunkte zur Fläche der optisch wirksamen Zone des optisch abbildenden Elements kleiner 8%, insbesondere kleiner 4% und besonders bevorzugt kleiner 2% ist. Dabei sei typischerweise die Gesamtfläche des optisch abbildenden Elements eine Fläche mit einem Durchmesser von 6mm, sowie die Fläche der optisch wirksamen Zone des optisch abbildenden Elements eine Zone, die durch die Pupillenöffnung bei normalen Lichtverhältnissen bestimmt wird, üblicherweise also eine Fläche mit einem Durchmesser von zirka 3mm.

Neben der Möglichkeit, das Nichtvorhandensein eines Markierungspunktes in einem Sektor bzw. einer Zelle zur Zustandsbeschreibung zu nutzen und damit Markierungspunkte "zu sparen", ist es generell zielführend, den Anteil der Fläche aller Markierungspunkte an der Gesamtfläche des optisch abbildenden Elements und insbesondere an der Fläche der optisch wirksamen Zone des optisch abbildenden Elements so gering wie nur möglich zu halten.

Das erfindungsgemäße codierte Punktraster aus Markierungspunkten ist also durch seine Struktur derart ausgelegt, dass es keine für den Patienten wahrnehmbare negative optische Wirkung hat. Um das zu gewährleisten, müssen die folgenden Bedingungen erfüllt sein:
a) Es hat einen pseudo-zufälligen, also unregelmäßigen Charakter, um Gitterbeugungseffekte zu minimieren (beispielsweise realisiert durch die versetzte Anordnung von Markierungspunkten zu einem polaren oder kartesischen Basisgitter),
b) die einzelnen Punkte sollten möglichst klein sein, und
c) es sollten so wenig Rasterpunkte wie für den Inhalt unbedingt nötig enthalten.

In einer weiteren vorteilhaften Ausgestaltung des erfindungsgemäßen ophthalmologischen Implantats weist das maschinenlesbare codierte Punktraster strukturelle Markierungspunkte auf. Strukturelle Markierungspunkte sind dabei Markierungspunkte, die durch eine Topologie gekennzeichnet sind, also physisch erhaben oder physisch eingeprägt sind, wobei letzteres die wahrscheinlichere Ausgestaltung dieser Markierungspunkte darstellt.

Vorteilhaft sind diese aus zwei Gründen: Zum einen gewährleisten diese Punkte noch eine gewisse Transparenz, sind also keine völlig undurchlässigen Absorber. Zum anderen ist die Erzeugung solcher strukturellen Markierungspunkte relativ leicht in den Fertigungsprozess eines ophthalmologischen Implantats einbindbar.

Nichtsdestotrotz ist jedoch auch eine Erzeugung von Markierungspunkten mittels Aufbringens bzw. Einbringens von Farbstoffen möglich, um das erfindungsgemäße ophthalmologische Implantat zu realisieren.

Der Kontrast von beispielsweise lasergravierten Markierungspunkten ist normalerweise durch Streuung gegeben. Bei neueren Technologien kann eine lokale optische Veränderung eingesetzt werden, um neben der Streuung auch Kontraste zu erzielen und die dargestellten Codes zu erzeugen:
- Es können Nanostrukturen eingesetzt werden, die als Lichtfallen wirken.
- Es können periodische Nanostrukturen zur Reflexion (von selektiven Wellenlängen) verwendet werden.
- Es können lokale Brechungsindexänderungen induziert werden, wie sie z.B. in lichtregulierbaren IOLs erzeugt werden.
- Es können organische und anorganische Farbstoffe und Absorber als Beschichtung oder lokal fixiert zum wellenlängenselektiven Auslesen des Codes genutzt werden. Als organischer Farbstoff im sichtbaren Bereich kann Dispersionsrot 1 verwendet werden, das durch Plasmaaktivierung auf der Oberfläche fixiert wird. Falls eine Auslesung im nahen IR-Bereich vorgesehen ist, können EpolightTM 1117 oder EpolightTM 1178 (beide von Epolin) lokal als Linsenbeschichtung aufgetragen werden. Ein Beispiel für anorganische Farbstoffe ist die Nutzung von Metall- oder Silikon-Nanopartikeln.
- Polychromatische Kombinationen von organischen und/oder anorganischen Farbstoffen und Absorbern können verwendet werden, um die Datendichte zu erhöhen, wodurch die Größe der Codes reduziert werden kann.
- Fotoaktivierbare Farbstoffe, die durch lokale laserinduzierte Lichtaktivierung in der Polymermatrix verankert werden können, sind ebenfalls möglich. Die Linse wird mit einer Lösung getränkt, die den Farbstoff (z. B. ein organisches Monomer wie Acryloxy-Fluorescein) und einen Initiator (falls erforderlich) enthält, was zu einer Diffusion in das Material führt, gefolgt von einer räumlichen Fotofixierung und einer Spülung der Linse, um nicht umgesetzten Farbstoff zu entfernen.
- Nicht-abrasive Methoden wie Druck oder Fotobleiche sind weitere Alternativen, um die gewünschten Marker zu erzeugen. Mikro-Tintenstrahlsysteme oder auch Mikro-Strukturierungs-Systeme können ebenfalls verwendet werden. So kann beispielsweise das derzeitige LUCIA-Heparin-Beschichtungsverfahren von Zeiss, das auf der fertigen Intraokularlinse angewendet wird, modifiziert und um einen Schritt erweitert werden, bei dem ein Farbstoffmuster kovalent an den Polymin-Linker auf der Linsenoberfläche gebunden wird.

Solche alternativen Ansätze sind vorteilhaft, da sie die Lichtmenge reduzieren, die von den Markierungspunkten gestreut wird und die Netzhaut erreicht und Nebenwirkungen wie "Star Bursts" oder andere Dysphotopsien verursacht. Bei wellenlängenselektiver Reflexion, Absorption oder Brechungsindexveränderungen sind möglicherweise zusätzliche Funktionen (chromatische Filter, Lichtpolarisatoren und -filter, Lampen) im optischen Auslesegerät erforderlich. Damit geht jedoch eine Reduktion von Nebenwirkungen auf den Seheindruck des Patienten einher. Zudem sind die Markierungen dann von außen nicht sichtbar sind (was dem Patienten kosmetisch zu Gute kommt). Dies erlaubt es wiederum, die codierten Marker problemlos in der zentralen Optik unterzubringen und einen leichteren optischen Zugang zu ermöglichen.

Weiterhin ist es von großem Vorteil, wenn das erfindungsgemäße ophthalmologische Implantat eine ergänzte Produktkennzeichnung enthält, die neben der ursprünglichen Produktkennzeichnung Informationen für Prüfsummen und Fehlerkorrekturverfahren aufweist.

Aufgrund der enormen Anzahl darstellbarer Zustände für eindeutige Produktkennzeichnungen besteht hinreichend Darstellungsreserve für Prüfsummen und Fehlerkorrekturmechanismen, die eine zusätzliche Sicherheit beim Einsatz eines solchen ophthalmologischen Implantats darstellen.

Die informationstheoretische Auslegung der Codierungsschemas erlaubt vorzugsweise die Codierung von sehr langen Ganzen Zahlen, um eine eindeutige Produktidentifizierung vieler (und verschiedener) Implantate über einen langen Zeitraum zu ermöglichen. Aufgrund der möglichen Speicherkapazität kann die Codierung selbst mit bekannten Verfahren aus der Informatik gegenüber Fehlauslesung abgesichert werden. Dazu zählen beispielsweise Prüfsummen- oder Fehlerkorrekturverfahren.

Zur Verbesserung der Erkennbarkeit und Referenzierung durch ein Maschinenlesesystem weist das ophthalmologische Implantat in einer weiteren Ausgestaltung eine oder mehrere Referenzmarken in einem definierten Abstand zum maschinenlesbaren codierten Punktraster aus Markierungspunkten auf.

Diese eine oder mehrere Referenzmarken sind also in der Umgebung des codierten Punktraster aus Markierungspunkten und in definierter Abstandsbeziehung zu diesem angeordnet und bilden die "Ansteuerpunkte" für ein Maschinenlesesystem, an dem sich dieses orientiert und hernach das codierte Punktraster aus Markierungspunkten in sehr einfacher Weise korrekt lokalisieren und auslesen kann.

Dabei kann in unterschiedlichen Ausgestaltungen des ophthalmologischen Implantats dessen maschinenlesbares codiertes Punktraster aus Markierungspunkten, bzw. ganz allgemein der codierte Marker, auf dem optisch abbildenden Element (also auf dessen Oberfläche aufgebracht bzw. in dessen Oberfläche eingebracht) oder im optisch abbildenden Element (also im Volumen des optisch abbildenden Elements) angeordnet sein. Auch eine Kombination beider Ausgestaltungen ist möglich.

Eine bevorzugte Ausführungsform des erfindungsgemäßen ophthalmologischen Implantats weist eine Ausrichtungshilfe auf. Die Ausrichtungshilfe ist ebenfalls vorzugsweise innerhalb der optisch wirksamen Zone angeordnet. Weiterhin vorteilhaft ist es, wenn die Ausrichtungshilfe das maschinenlesbare codierte Punktraster aus Markierungspunkten enthält oder aus diesem besteht.

Eine weitere bevorzugte Ausführungsform des erfindungsgemäßen ophthalmologischen Implantats weist einen torischen Marker, der in der Ansicht von oben lesbar ist, und/oder einen torischen Marker, der in einer axialen Ansicht lesbar ist, auf. Der torische Marker kann ebenfalls auf der Oberfläche des optisch abbildenden Elements und/oder in dessen Volumen angeordnet sein. Außerdem kann auch der torische Marker das maschinenlesbare codierte Punktraster aus Markierungspunkten enthalten oder aus diesem bestehen.

Ein torischer Marker, der in der Ansicht von oben lesbar ist, erlaubt einen Zugang über eine Kamera in der Produktion und in der Logistik und über ein Operationsmikroskop, eine Spaltlampe oder ein Biometriegerät im implantierten Zustand ermöglicht. Ein torischer Marker, der in axialer Ansicht (also in Seitenansicht) lesbar ist, ist beispielsweise tomographischen Messungen zugänglich. Er kann zum Beispiel mit OCT (Optischer Kohärenztomographie), CT (Computertomographie) oder MRI (Magnetresonanztomographie) genutzt werden.

Daten-Matrix-Codes in Form eines torischen Markers dienen zur Unterstützung der In-vivo-Linsenausrichtung im Auge während der Operation und der Rückverfolgbarkeit der Rotation. Zur Korrektur eines regelmäßigen Astigmatismus weisen torische Intraokularlinsen einen optischen Zylinder auf, der entlang einer bestimmten Achse, der torischen Achse, angeordnet ist. Die torische Achse wird durch die torischen Marker angezeigt. Da die Ausrichtung der Linse im Auge während der Operation mit dem Operationsmikroskop über eine Software wie Callisto erfolgt, können die erforderlichen Linseninformationen in vivo automatisch geliefert werden. Darüber hinaus ist diese Funktion nicht nur für torische IOLs, sondern auch für monofokale IOLs vorgesehen, was eine operative in-vivo und postoperative Verfolgung der Linsenzentrierung und -rotation ermöglicht. Dabei dient der Data-Matrix-Code als optische Referenz, um die Linsenausrichtung in vivo zu verfolgen.

Hier werden also verschiedene Ausführungsformen von codierten Markern vorgestellt, die sich von einem Standardcode unterscheiden. Insbesondere ist der torische Marker sowohl in Standardroutinen (biometrische Geräte, chirurgische Mikroskope, Spaltlampen) und/oder in fortgeschritteneren optischen Designs (Pinhole-Konzepte, zentraloptische Designs) visuell zugänglich.

Die Ausführungsformen mit Ausrichtungshilfe und/oder torischen Marker für alle ophthalmologischen Implantate, insbesondere für Intraokularlinsen (torisch und nichttorisch; modulare Linsenausrichtung), ermöglichen es auch, postoperative Änderungen der Implantat-Position (Neigung, Dezentrierung, Drehung aller Linsen) zu detektieren.

Bevorzugt ist für das maschinenlesbare codierte Punktraster aus Markierungspunkten, bzw. ganz allgemein dem codierten Marker, eine Markierungspunktgröße (Spot-Größe) von ~25µm, basierend auf Tests an einer in ein menschliches Augenmodell implantierten Intraokularlinse. Sie stellt den besten Kompromiss zwischen Größe und Kontrast dar, der im Operationsmikroskop sichtbar ist. Für OCT A ist eine konventionelle axiale Auflösung von ~5µm möglich. Dies wäre eine untere Grenze der Markierungspunktgröße, über Auflösungskriterien könnte ein Faktor von 2 angewendet werden.

Bei einem Biomaterialcode (wie also hier einem codierten Marker auf einem ophthalmologischen Implantat) ist es von Vorteil, die Rückverfolgbarkeit über die gesamte Lebensdauer des Implantats zu ermöglichen. Ein größerer Abstand zwischen den Markierungspunkten (Gittergröße > Punktgröße) verringert mögliche Beugungseffekte. Die Verwendung nicht-periodischer Datencodes, nicht nur auf dem Niveau der Anordnung einzelner Markierungspunkte, sondern auch bezüglich der Gesamtstruktur der codierten Marker, ist von Vorteil. Kontrastverbesserungen wie laserinduzierte Lichtfallen oder reflektierende Strukturen oder Brechungsindexänderungen sind ebenfalls von Vorteil, um das Risiko einer Dysphotopsie zu verringern.

Ein erfindungsgemäßes Maschinenlesesystem dient der Erfassung und Decodierung der digitalen Produktkennzeichnung in Form eines codierten Punktrasters aus Markierungspunkten, bzw. allgemein des codierten Markers, auf einem hier beschriebenen ophthalmologischen Implantat.

Das Maschinenlesesystem umfasst ein Kamerasystem zur Aufnahme der Strukturen des maschinenlesbaren codierten Punktrasters aus Markierungspunkten auf dem ophthalmologischen Implantat, eine Analyseeinheit zur Erfassung und Auswertung eines vom Kamerasystem aufgenommenen Bildes der Strukturen des maschinenlesbaren codierten Punktrasters aus Markierungspunkten sowie zur Decodierung der digitalen Produktkennzeichnung des ophthalmologischen Implantats, insbesondere beispielsweise des Typs und der Brechkraft und/oder des Datenbankschlüssels, zu dessen Identifikation aus diesem Bild.

In Abhängigkeit von den jeweiligen Lichtverhältnissen kann ein solches Maschinenlesesystem ein Beleuchtungssystem zur Beleuchtung einer digitalen Produktkennzeichnung einer Intraokularlinse in Form eines maschinenlesbaren codierten Punktrasters aus Markierungspunkten aufweisen.

Vorteilhaft ist es des Weiteren, wenn das Maschinenlesesystem auch eine Anzeige- und/oder Ausgabevorrichtung zur Anzeige und/oder Ausgabe der decodierten Identifikationsdaten des ophthalmologischen Implantats umfasst. Andernfalls kann jedoch eine solche Anzeige bzw. Ausgabe auch von anderen Vorrichtungen, die mit dem Maschinenlesesystem verbunden werden können, übernommen werden.

Insbesondere, wenn die digitale Produktkennzeichnung des ophthalmologischen Implantats einen Datenbankschlüssel enthält, ist es des Weiteren vorteilhaft, wenn das Maschinenlesesystem mit einem Datenbanksystem verbunden ist. Durch dieses Datenbanksystem werden einem Datenbankschlüssel, der ein eindeutiger Identifier ist, die implantatspezifischen Produktinformationen zugeordnet. Durch das Auslesen des als digitale Produktkennzeichnung des ophthalmologischen Implantats hinterlegten Datenbankschlüssels durch das Maschinenlesesystem kann eine Datenbankabfrage von implantatspezifischen Produktinformationen durchgeführt werden. Mittels dieser Vorgehenswese können bei kleinen Kodierungslängen umfangreiche und eindeutige Produktinformationen zugänglich gemacht werden. Das Datenbanksystem wird vorzugsweise in einem Datennetzwerk oder einer Datencloud zur Verfügung gestellt. In diesem Datenbanksystem hinterlegt beispielsweise der Hersteller zu jedem eindeutigen Identifier die implantatspezifischen Produktinformationen.

In konkreten Ausführungsformen ist das erfindungsgemäße Maschinenlesesystem Teil eines Operationsmikroskops oder einer Spaltlampe.

Die eindeutige Produktkennzeichnung kann dabei vom Maschinenlesesystem gespeichert und/oder verarbeitet sowie für weitere Dienste digital zur Verfügung gestellt werden. Ein solches Maschinenlesesystem kann sich dabei in der Produktion des ophthalmologischen Implantats, beispielsweise in einer Produktion von Intraokularlinsen zur Qualitätsüberwachung und/oder in der Praxis des implantierenden und/oder kontrollierenden Augenarztes zur Überprüfung des ophthalmologischen Implantats befinden.

In einem erfindungsgemäßen Verfahren zur Herstellung eines ophthalmologischen Implantats mit einer digitalen Kennzeichnung wie oben beschrieben wird das maschinenlesbare codierte Punktraster aus Markierungspunkten zur digitalen Produktkennzeichnung während oder nach der Herstellung des ophthalmologischen Implantats auf diesem erzeugt.

Die Kennzeichnung wird also während des Herstellungsprozesses mit dem ophthalmologischen Implantat, insbesondere mit einer Intraokularlinse (IOL), verbunden und kann dann während weiterer Folgeschritte bei der Kataraktoperation und im implantierten Zustand mit optischen Mitteln ausgelesen, gespeichert und verarbeitet werden.

In einer bevorzugten Ausgestaltung des erfindungsgemäßen Verfahrens zur Herstellung eines ophthalmologischen Implantats wird dieses während der Herstellung des ophthalmologischen Implantats
- entweder in einer frühen Phase, also vor dem Abschluss der Herstellung des ophthalmologischen Implantats, mit dem maschinenlesbaren codierten Punktraster aus Markierungspunkten gekennzeichnet,
- oder direkt nach dem Abschluss der Herstellung, aber noch in denselben Schritt mit grundsätzlich demselben Werkzeug, mit dem die Herstellung des ophthalmologischen Implantats erfolgt ist, mit dem maschinenlesbaren codierten Punktraster aus Markierungspunkten gekennzeichnet.

Besonders bevorzugt ist eine Ausgestaltung des erfindungsgemäßen Verfahrens zur Herstellung eines ophthalmologischen Implantats, in dem das maschinenlesbare codierte Punktraster aus Markierungspunkten mittels eines CNC gesteuerten Bohr- bzw. Fräswerkzeugs in die Oberfläche während oder nach der Herstellung des ophthalmologischen Implantats in dieses eingebracht wird, wobei herfür vorzugsweise das Bohr- bzw. Fräswerkzeug einen Werkzeugdurchmesser von kleiner 0,4mm aufweist.

Üblicherweise werden spanabhebenden Drehverfahren, insbesondere Diamantdrehverfahren, zur Herstellung von ophthalmologischen Implantaten selbst eingesetzt, also hierzu CNC-gesteuerte Fräswerkzeuge genutzt. Idealerweise wird nun das ophthalmologische Implantat digital gekennzeichnet, ohne dass es hierfür das Werkzeug wechseln muss, da jeder Maschinenwechsel und damit Ortswechsel des ophthalmologischen Implantats natürlich eine Quelle von Verwechslungen sein kann. Kann jedoch das ophthalmologische Implantat mit prinzipiell demselben Werkzeug digital gekennzeichnet werden, mit dem es auch hergestellt wird, so ist ein Verfahren möglich, indem die Daten, die aktiv während des Herstellungsprozesses "gesammelt" werden bzw. im Herstellungsprozess des ophthalmologischen Implantats eingesetzt werden, auch während oder direkt nach der Herstellung in dieses ophthalmologische Implantat codiert werden. Eine Verwechslung des Datensatzes oder des ophthalmologischen Implantats ist damit ausgeschlossen.

Die spanende Bearbeitung zur digitalen Produktkennzeichnung erfolgt dabei mit kleinen Werkzeugen (vorzugsweise mit einem Durchmesser < 0.4mm) in einem oberflächennahen Bereich.

**In** einer alternativen Ausgestaltung des erfindungsgemäßen Verfahrens zur Herstellung eines ophthalmologischen Implantats wird das maschinenlesbare codierte Punktraster aus Markierungspunkten entweder mittels Laserbearbeitung durch Ablation oder Disruption oder mittels Druckverfahren, vorzugsweise mit biokompatiblen Chromophoren oder Pigmenten, welche sich üblicherweise in einer kovalent an das Linsenmaterial bindenden Matrix befinden, aufgebracht.

**In** einer Ausgestaltung des erfindungsgemäßen Verfahrens zur Herstellung eines ophthalmologischen Implantats, wird dabei eine Produktkennzeichnung oder aber eine ergänzte Produktkennzeichnung in Rasterkoordinaten für die physische Produktkennzeichnung mittels maschinenlesbarem codierten Punktraster aus Markierungspunkten umgerechnet.

Die Verbindung der eindeutigen Produktkennzeichnung mit dem ophthalmologischen Implantat erfolgt mittels des erfindungsgemäßen Punktrasters aus Markierungspunkten. Dazu wird die eindeutige Produktkennzeichnung zunächst, sofern dies vorgesehen, um die Informationen für Prüfsummen- oder Fehlerkorrekturverfahren ergänzt. Die genaue Vorgehensweise hängt hierbei vom ausgewählten Verfahren ab. Danach wird die ergänzte eindeutige Produktkennzeichnung in die Rasterkoordinaten für die physische Produktkennzeichnung umgerechnet. Anhand der Rasterkoordinaten kann nun die Kennzeichnung auf das Produkt übertragen werden in das Punktraster aus Markierungspunkten.

Nicht zuletzt wird in einer weiteren Ausgestaltung des Verfahrens während oder nach der Erzeugung des maschinenlesbaren codierten Punktrasters aus Markierungspunkten zur digitalen Produktkennzeichnung dieses in einem Datenbanksystem des Herstellers gespeichert, das mit einer elektronischer Patientenakte und/oder einer andern Datensammelstelle für ärztliche oder behördliche Zwecke verbunden werden kann.

Die eindeutige Produktkennzeichnung wird in der Regel durch den Hersteller selbst oder ggf. mittels einer Zertifizierungsstelle während der Herstellung des ophthalmologischen Implantats erzeugt. Diese Kennzeichnung wird dann erfindungsgemäß mit dem Produkt verbunden und in einem Datenbanksystem gespeichert. Dieses Datenbanksystem kann geschlossen beim Hersteller und/oder bei einer öffentlichen Datensammelstelle für ärztliche oder behördliche Zwecke angelegt sein bzw. vom Hersteller zu einer entsprechenden Datensammelstelle übertragen werden. Auch in einer elektronische Patientenakte kann diese Produktkennzeichnung abgelegt werden.

In einer wichtigen Ausgestaltung des erfindungsgemäßen Verfahrens zur Herstellung eines ophthalmologischen Implantats werden vor der Erzeugung des maschinenlesbaren codierten Punktrasters aus Markierungspunkten zur digitalen Produktkennzeichnung in einem Datenbanksystem die Produktinformationen des ophthalmologischen Implantats hinterlegt und ein Datenbankschlüssel zu diesen Produktinformationen, der in der digitalen Produktkennzeichnung enthalten ist, wird generiert.

Ein solcher Verfahrensschritt kann anstelle des zuvor zitierten Verfahrensschrittes der Speicherung der digitalen Produktkennzeichnung in einem Datenbanksystem während oder nach der Erzeugung erfolgen. Es ist jedoch auch möglich, dass vor der Erzeugung ein Datenbankschlüssel im Datenbanksystem generiert wird und während oder nach der Erzeugung wiederum schreibend auf das Datenbanksystem zugegriffen wird, um die tatsächlich erzeugte digitale Produktkennzeichnung, insbesondere den Datenbankschlüssel, der durch das maschinenlesbare codierte Punktraster als Markierungspunkten auf dem ophthalmologischen Implantat erzeugt wurde, zu bestätigen. Dabei findet vorteilhafterweise ein Abgleich statt, und bei Abweichungen bzgl. des Datenbankschlüssels wird ein fehlerhaft gekennzeichnetes ophthalmologisches Implantat gesperrt.

Nachfolgend wird die Erfindung beispielhaft anhand der beigefügten Zeichnungen, die auch erfindungswesentliche Merkmale offenbaren, noch näher erläutert. Es zeigen:
- die Fig. 1 eine digitale Produktkennzeichnung, wie sie in einem erfindungsgemäßen ophthalmologischen Implantat, hier im ersten Ausführungsbeispiel der Fig. 2 und 2a, verwendet werden kann, die Fig. 1a einen vergrößerten Ausschnitt dieser digitalen Produktkennzeichnung;
- die Fig. 2 ein erstes Ausführungsbeispiel eines erfindungsgemäßen ophthalmologischen Implantats, hier eine Intraokularlinse, die Fig. 2a eine vergrößerte Abbildung der Produktkennzeichnung auf dem optisch abbildenden Element, hier auf dem Linsenkörper einer Intraokularlinse;
- die Fig. 3 ein zweites Ausführungsbeispiel eines erfindungsgemäßen ophthalmologischen Implantats, die Fig. 3a eine vergrößerte Abbildung der eine vergrößerte Abbildung der Produktkennzeichnung auf dem optisch abbildenden Element;
- die Fig. 4 ein drittes Ausführungsbeispiel eines erfindungsgemäßen ophthalmologischen Implantats, die Fig. 4a eine vergrößerte Abbildung der eine vergrößerte Abbildung der Produktkennzeichnung auf dem optisch abbildenden Element;
- die Fig. 5 ein viertes Ausführungsbeispiel eines erfindungsgemäßen ophthalmologischen Implantats, die Fig. 5a eine vergrößerte Abbildung der eine vergrößerte Abbildung der Produktkennzeichnung auf dem optisch abbildenden Element;
- die Fig. 6 ein fünftes Ausführungsbeispiel eines erfindungsgemäßen ophthalmologischen Implantats, die Fig. 6a eine vergrößerte Abbildung des zur digitalen Produktkennzeichnung genutzten maschinenlesbaren Punktrasters aus Markierungspunkten;
- die Fig. 7 ein sechstes Ausführungsbeispiel eines erfindungsgemäßen ophthalmologischen Implantats, die Fig. 7a eine vergrößerte Abbildung des zur digitalen Produktkennzeichnung genutzten maschinenlesbaren Punktrasters aus Markierungspunkten;
- die Fig. 8 eine Intensitätsverteilung unmittelbar hinter einer mit dem codierten Punktraster aus Markierungspunkten des sechsten Ausführungsbeispiels markierten Fläche;
- die Fig. 9 die Modulationsübertragungsfunktion für das zentrale Punktraster aus Markierungspunkten des sechsten Ausführungsbeispiels;
- die Fig. 10 ein erfindungsgemäßes Maschinenlesesystem zur Erfassung und Decodierung eines codierten Punktrasters aus Markierungspunkten auf einem ophthalmologischen Implantat.
- die Fig. 11 die Nutzung eines codierten Markers zu einer Verifizierung des Implantats während der Implantation und in-vivo nach der Implantation;
- die Fig. 12a bis 12c eine digitale Produktkennzeichnung mit torischem Marker und Ausrichtungshilfe nach dem Stand der Technik und in verschiedenen Ausführungsbeispielen des erfindungsgemäßen ophthalmischen Implantats;
- die Fig. 13a bis 13c digitale Produktkennzeichnungen mit torischem Marker in der optisch wirksamen Zone des optisch abbildenden Elements in weiteren verschiedenen Ausführungsbeispielen des erfindungsgemäßen ophthalmischen Implantats;
- die Fig. 14a bis 14c eine digitale Produktkennzeichnung mit torischem Marker in weiteren verschiedenen Ausführungsbeispielen des erfindungsgemäßen ophthalmischen Implantats - für die Nutzung aus der Ansicht von oben sowie für die Nutzung in axialer Ansicht;
- die Fig. 15a bis 15c eine digitale Produktkennzeichnung in weiteren verschiedenen Ausführungsbeispielen des erfindungsgemäßen ophthalmischen Implantats;
- die Fig. 16 verschiedene Anordnungen des maschinenlesbaren codierten Punktrasters aus Markierungspunkten einer digitalen Produktkennzeichnung;
- die Fig. 17a und 17b eine Größenabschätzung für M3 bzw. M4 UDI Codes;
- die Fig. 18a bis 18d ein Herstellungsverfahren einer Intraokularlinse mit einem codierten Marker der im Volumen des optisch abbildenden Elements angeordnet ist;
- die Fig. 19a bis 19c eine in ein ISO-Auge implantierte Intraokularlinse mit einem codierten Marker auf der Oberfläche des optisch abbildenden Elements und den Halo/Glare Test mit diesem und ohne diesen codierten Marker.

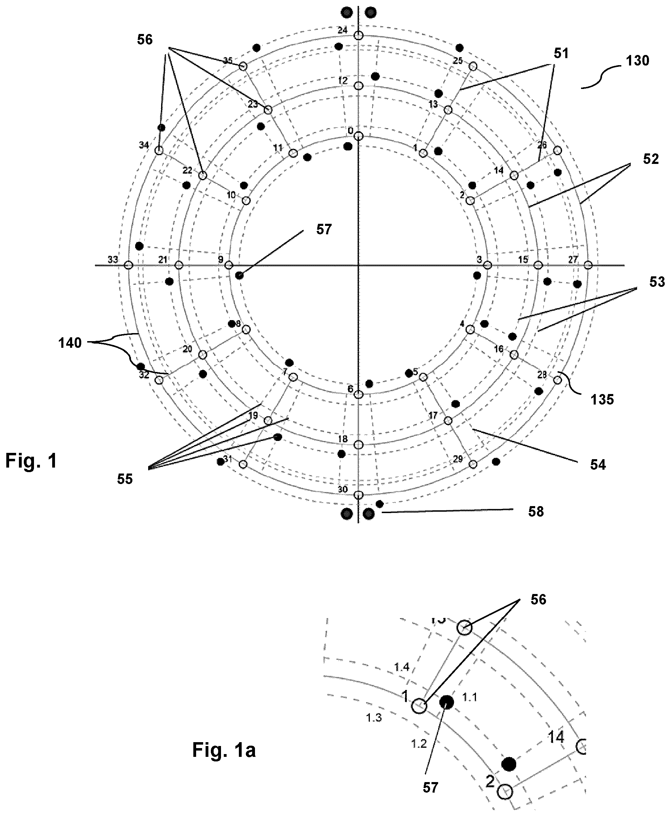

In der Fig. 1 ist zunächst eine digitale Produktkennzeichnung 130 dargestellt, wie sie in einem erfindungsgemäßen ophthalmologischen Implantat 100 verwendet werden kann, um dessen Prinzipien besser erläutern zu können. Die Fig. 1a zeigt einen vergrößerten Ausschnitt dieser digitalen Produktkennzeichnung 130. Das (maschinenlesbare) codierte Punktraster 135 aus Markierungspunkten 57 zur digitalen Produktkennzeichnung 130, das in den Fig. 1 und 1a gezeigt wird, wird durch die versetzte Anordnung der Markierungspunkte 57 zu einem polaren Basisgitter erzielt, wobei die Erläuterungen jedoch prinzipiell auch für eine Anordnung von Markierungspunkten 57 in einem kartesischen Basisgitter gelten (in dem ein Sektor 51 dann Zelle 51' genannt wird).

Das hier gezeigt polares Basisgitter besteht aus drei radialen Zonen 52 mit jeweils zwölf Sektoren 51. Diese erzeugen die Basisgitterpunkte 56, die allein virtueller Natur sind. Die Basisgitterpunkte sind in der Fig. 1 fortlaufend von 0 bis 35 indiziert und kennzeichnen den jeweiligen Sektor 51 (bzw. die jeweilige Zelle). Darüber hinaus enthält das Gitter in sektoraler 54 und radialer 53 Richtung Zonen mit positivem und negativem Versatz. Diese erzeugen um die Basisgitterpunkte 56 herum vier weitere Kreuzungspunkte 55. Auf einem dieser vier Kreuzungspunkte 55 kann sich des Markierungspunkt 57 befinden - die vier Kreuzungspunkte stellen also die möglichen Positionen des Markierungspunktes 57 in dem entsprechenden Sektor 51 bzw. in der entsprechenden Zelle 51' dar.

Die Fig. 1a zeigt dann eine vergrößerte Ansicht eines Basisgitterpunkts 56 und seiner Umgebung bzw. seines entsprechenden Sektors 51. Darin ist ersichtlich, dass die vier Positionen 55 um den Basisgitterpunkt 56 indiziert sind. Für den Basisgitterpunkt 56 mit dem Index 1 sind das die Positionen 1.1, 1.2, 1.3 und 1.4. In diesem Beispiel kann der zum Basisgitterpunkt 56 gehörige Markierungspunkt 57 des Sektors 51 eine der vier verschiedenen Positionen 55 1.1, 1.2, 1.3 oder 1.4 einnehmen. Somit kann in diesem Beispiel ein einzelner Basisgitterpunkt 56 vier Zustände einnehmen. Bei insgesamt 36 Sektoren mit jeweils vier Positionen 55 (also vier möglichen Zuständen) erhält man somit 4³⁶ = 4 722 366 482 869 645 213 696 darstellbare Zustände.

Zu Verbesserung der Erkennbarkeit und Referenzierung durch ein Maschinenlesesystem 200 sind zudem mehrere Referenzmarken 58 in der Umgebung des Punktrasters 135 aus Markierungspunkten 57 angeordnet.

Im hier erläuterten Beispiel der Fig. 1 kann der jeweilige Markierungspunkt 57 eine von vier möglichen Positionen 55 um einen Basispunkt 56 einnehmen. Somit werden insgesamt 36 Markierungspunkte 57 in dem polaren Basisgitter angeordnet.

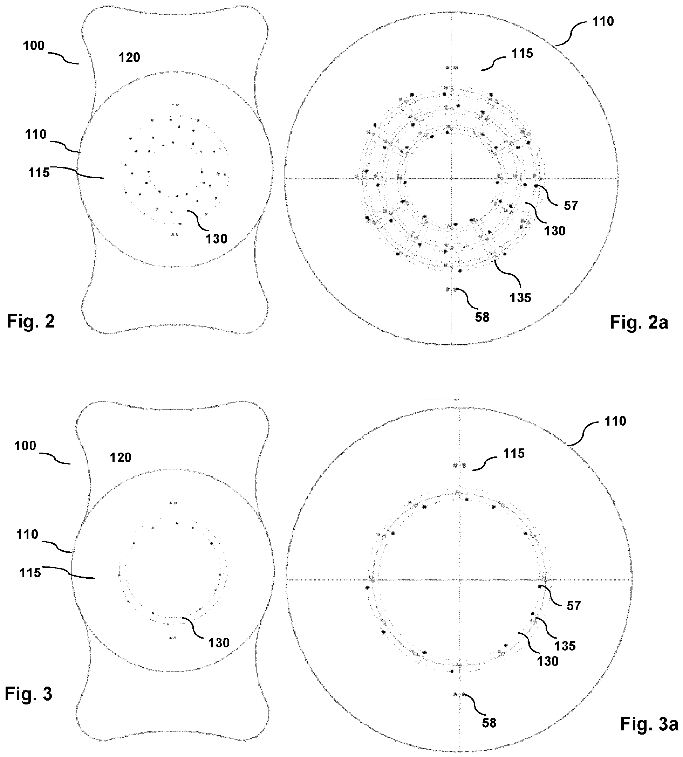

Die Fig. 2 zeigt ein erstes Ausführungsbeispiel eines erfindungsgemäßen ophthalmologischen Implantats 100, hier eine Intraokularlinse, mit einer digitalen Produktkennzeichnung 130, die Fig. 2a eine vergrößerte Abbildung der Produktkennzeichnung 130 auf dem optisch abbildenden Element 110, hier auf dem Linsenkörper einer Intraokularlinse. Bei diesem ersten Ausführungsbeispiel handelt es sich um ein codiertes Punktraster 135 aus Markierungspunkten 57, das ein polares Basisgitter nutzt und dabei drei Zonen 52 und zwölf Sektoren 51 pro Zone 52 mit jeweils vier Zuständen pro Sektor 51 aufweist. Damit ergeben sich, wie im Beispiel der Fig. 1 gezeigt, 4³⁶ darstellbare Zustände, die zum Hinterlegen der digitalen Produktinformation genutzt werden können. In diesem Ausführungsbeispiel beträgt die Einzelpunkt-Größe eines Markierungspunktes 57 ungefähr 0,0025mm², damit liegt der Anteil der Fläche der Markierungspunkte 57 zur Gesamtfläche des optisch abbildenden Elements 110 bei 0,3178 %.

Die Fig. 3 zeigt ein zweites Ausführungsbeispiel eines erfindungsgemäßen ophthalmologischen Implantats 100, die Fig. 3a eine vergrößerte Abbildung der Produktkennzeichnung 130 auf dem optisch abbildenden Element 110 dieses ophthalmologischen Implantats 100. Bei diesem zweiten Ausführungsbeispiel handelt es sich um ein codiertes Punktraster 135 aus Markierungspunkten 57, das ein polares Basisgitter nutzt und dabei eine Zone 52 mit zwölf Sektoren 51 aufweist, wobei jeder Sektor vier Zustände beschreiben kann. Somit erhält man 4¹² = 16 777 216 darstellbare Zustände.

In diesem Ausführungsbeispiel beträgt die Einzelpunkt-Größe eines Markierungspunktes 57 ungefähr 0,0025mm². Damit liegt der Anteil der Fläche der Markierungspunkte 57 zur Gesamtfläche des optisch abbildenden Elements 110 bei 0,1059 %.

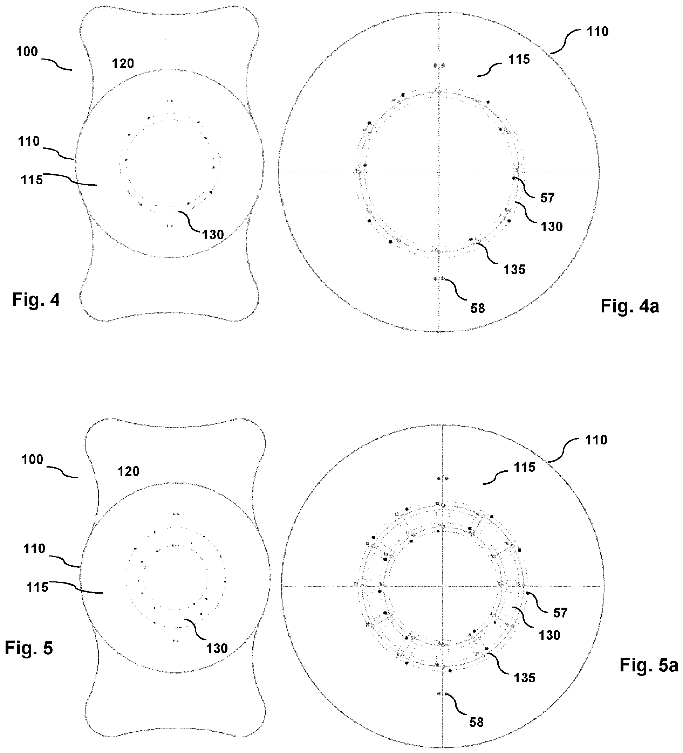

**In** der Fig. 4 ist ein drittes Ausführungsbeispiel eines erfindungsgemäßen ophthalmologischen Implantats 100, in der Fig. 4a eine vergrößerte Abbildung der digitalen Produktkennzeichnung 130 auf dem optisch abbildenden Element 110 dargestellt. Auch bei diesem dritten Ausführungsbeispiel handelt es sich um ein polares Basisgitter mit einer Zone 52, die zwölf Sektoren 51 aufweist. Durch jeden dieser Sektoren 51 können hier jedoch fünf Zustände beschrieben werden, da zusätzlich zu den vier möglichen Zuständen durch die Lage eines Markierungspunktes 57 auf einer der vier Positionen 55, die einen Versatz zum Basisgitterpunkt 56 aufweisen, auch das Nichtvorhandensein eines Markierungspunktes 57 in dem entsprechenden Sektor 51 einen weiteren Zustand beschreibt. Somit erhält man 5¹² = 244 140 625 darstellbare Zustände.

In diesem Ausführungsbeispiel beträgt die Einzelpunkt-Größe eines Markierungspunktes 57 wiederum ungefähr 0,0025mm², damit liegt der Anteil der Fläche der Markierungspunkte 57 zur Gesamtfläche des optisch abbildenden Elements 110 bei 0,1059 %. Obwohl sich also die mögliche Speicherkapazität erhöht hat, ist der Anteil der Fläche der Markierungspunkte 57 zur Gesamtfläche des optisch abbildenden Elements 110 im Vergleich zum zweiten Ausführungsbeispiel gleichgeblieben.

Die Fig. 5 zeigt ein viertes Ausführungsbeispiel eines erfindungsgemäßen ophthalmologischen Implantats 100, die Fig. 5a wiederum eine vergrößerte Abbildung der Produktkennzeichnung 130 auf dem optisch abbildenden Element 110 des ophthalmologischen Implantats 100. Bei diesem vierten Ausführungsbeispiel handelt es sich wiederum um ein polares Basisgitter, jedoch mit zwei Zonen 52, die jeweils zwölf Sektoren 51 aufweisen. Durch jeden dieser Sektoren 51 können auch hier fünf Zustände beschrieben werden. Somit erhält man 5¹² = 59 604 644 775 390 625 darstellbare Zustände.

In diesem Ausführungsbeispiel beträgt die Einzelpunkt-Größe eines Markierungspunktes 57 wiederum ungefähr 0,0025mm², damit liegt der Anteil der Fläche der Markierungspunkte 57 zur Gesamtfläche des optisch abbildenden Elements 110 bei 0,2119 %.

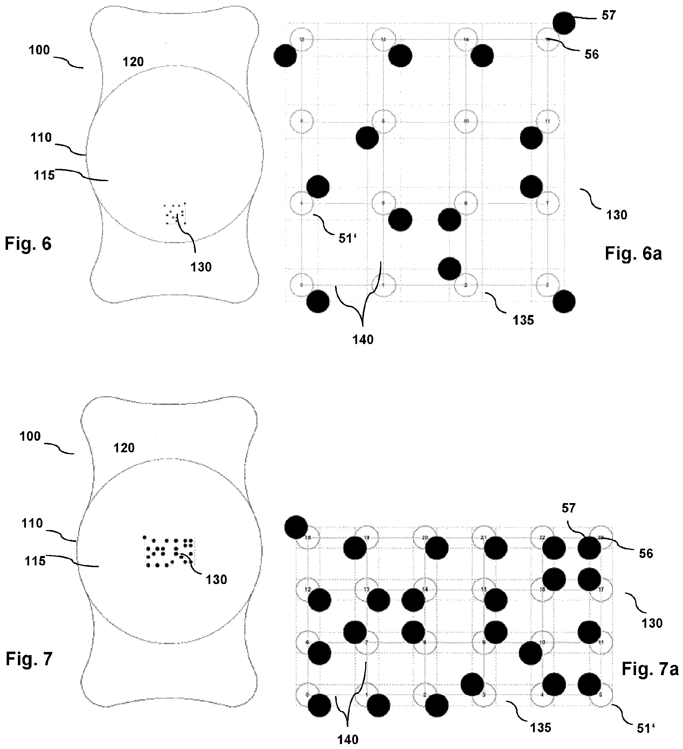

Die Fig. 6 zeigt ein fünftes Ausführungsbeispiel eines erfindungsgemäßen ophthalmologischen Implantats 100, die Fig. 6a eine vergrößerte Abbildung des zur digitalen Produktkennzeichnung 130 genutzten maschinenlesbaren Punktrasters 135 aus Markierungspunkten 57. Bei diesem fünften Ausführungsbeispiel wird jedoch ein kartesisches Basisgitter genutzt mit einer Ausdehnung von vier Zellen 51' in eine laterale Richtung x und vier Zellen 51' in eine laterale Richtung y (also nₓ=4, n_{y}=4), in dem durch jede Zelle 51' jeweils fünf Zustände beschrieben werden können, also vier Zustände durch die Lage eines Markierungspunktes 57 auf einer der vier möglichen Positionen 55, die einen Versatz (in jeweils unterschiedlicher Richtung) zum Basisgitterpunkt 56 der Zelle 51' aufweisen, sowie einen zusätzlichen Zustand durch das Nichtvorhandensein eines Markierungspunktes 57 4¹⁶ = 4 294 967 296 darstellbare Zustände. Auch in diesem Ausführungsbeispiel beträgt die Einzelpunkt-Größe eines Markierungspunktes 57 ungefähr 0,0025mm². Damit liegt der Anteil der Fläche der Markierungspunkte 57 zur Gesamtfläche des optisch abbildenden Elements 110 bei 0,1413 %.

In der Fig. 7 ist ein sechstes Ausführungsbeispiel eines erfindungsgemäßen ophthalmologischen Implantats 100, wiederum eine Intraokularlinse, und in der Fig. 7a eine vergrößerte Abbildung des zur digitalen Produktkennzeichnung 130 genutzten maschinenlesbaren Punktrasters135 aus Markierungspunkten 57 dargestellt. Auch in diesem sechsten Ausführungsbeispiel wird ein kartesisches Basisgitter genutzt mit einer Ausdehnung von sechs Zellen 51' in eine laterale Richtung x und vier Zellen 51' in eine laterale Richtung y (nx = 6, ny =4), in dem durch jede Zelle 51' jeweils vier Zustände beschrieben werden können. Damit stehen hier 4²⁴ = 281 474 976 710 656 darstellbare Zustände zum Speichern der (ggf. ergänzten) Produktkennzeichnung 130 zur Verfügung. in diesem Ausführungsbeispiel beträgt die Einzelpunkt-Größe eines Markierungspunktes 57 ungefähr 0,0113mm². Damit liegt der Anteil der Fläche der Markierungspunkte 57 zur Gesamtfläche des optisch abbildenden Elements 110 bei 0,96 %, und damit deutlich höher als in den vorangegangenen Ausführungsbeispielen.

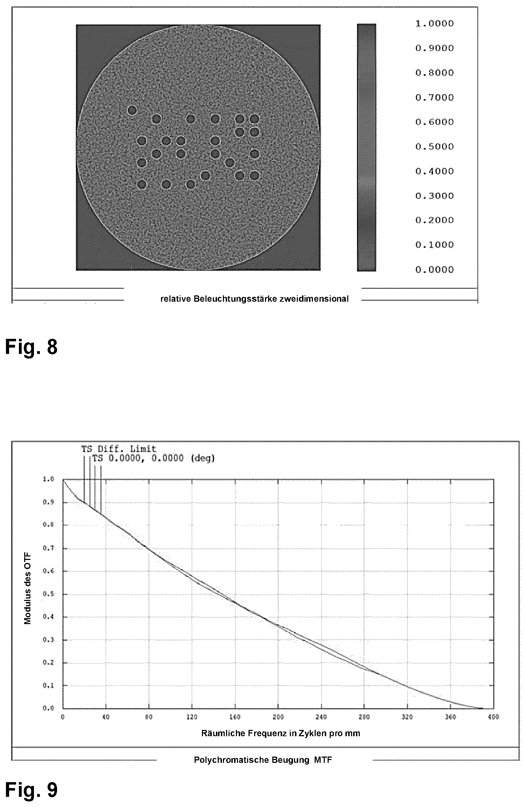

Im sechsten Ausführungsbeispiel der Fig. 7 und 7a (Kartesisches Basisgitter, nₓ = 6, n_{y} =4, vier Zustände) ist als optisches Worst-case - Beispiel eine kartesische Punktraster 135 aus Markierungspunkten 57 mit verhältnismäßig großen Markierungspunkten 57 im Zentrum einer Intraokularlinse dargestellt. Dieses Muster wurde als "User Defined Obscuration" in ein Augenmodell eines Simulationsprogramms (ZEMAX) importiert und die Modulationsübertragungsfunktion (MTF) auf der Retina bestimmt. Dabei wurde eine kleine Pupille vom Durchmesser 3.0mm gewählt, um einen möglichst hohen Störanteil des Musters innerhalb der Pupille zu erreichen. Die Markierungspunkte 57 sind in diesem Beispiel völlig undurchlässige Absorber - in der Praxis z.B. schwarze Punkte. Die Fig. 8 zeigt die Intensitätsverteilung unmittelbar hinter der markierten Fläche. Das Muster entspricht genau dem Beispiel aus den Fig. 7 und 7a. Die Fig. 9 zeigt jedoch, dass die MTF durch das zentrale Punktraster praktisch unbeeinflusst ist und nahe an der Beugungsgrenze verbleibt.

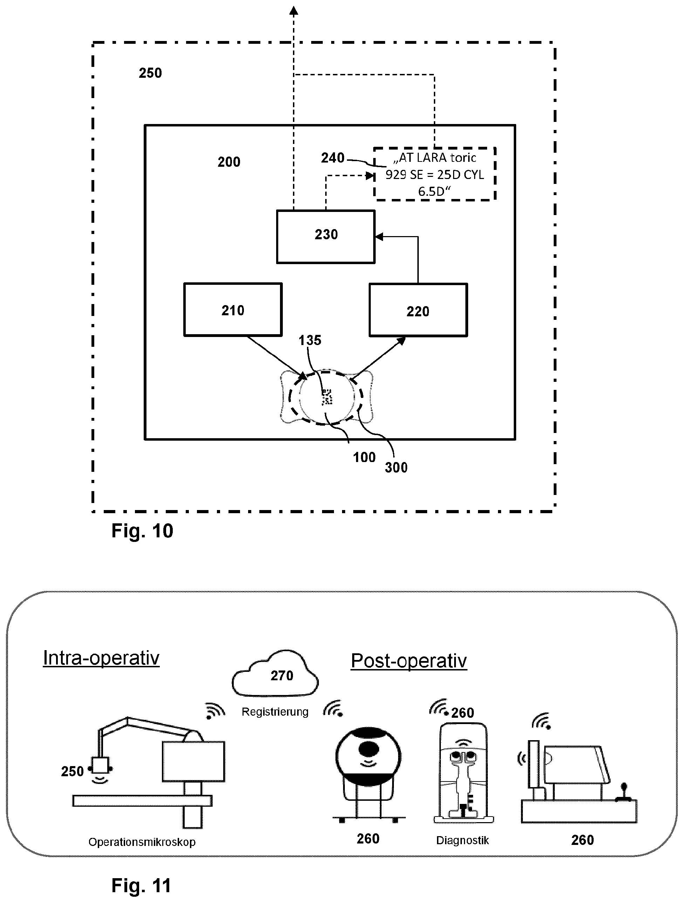

In der Fig. 10 ist schließlich ein erfindungsgemäßes Maschinenlesesystem 200 zur Erfassung und Decodierung eines codierten Punktrasters aus Markierungspunkten auf einem ophthalmologischen Implantat 100, insbesondere auf einer Intraokularlinse, dargestellt, das Teil eines entsprechenden Operationsmikroskops 250 ist.

Dieses Ausführungsbeispiel eines erfindungsgemäßen Maschinenlesesystems 200 weist ein Beleuchtungssystem 210 zur Beleuchtung einer digitalen Produktkennzeichnung 130 einer Intraokularlinse in Form eines maschinenlesbaren codierten Punktrasters aus Markierungspunkten 135, ein Kamerasystem 220 zur Aufnahme von mittels der Beleuchtung detektierbar gemachten Strukturen des maschinenlesbaren codierten Punktrasters aus Markierungspunkten 135 auf der Intraokularlinse, eine Analyseeinheit 230 zur Erfassung und Auswertung eines vom Kamerasystem 220 aufgenommenen Bildes der mittels der Beleuchtung detektierbar gemachten Strukturen des maschinenlesbaren codierten Punktrasters aus Markierungspunkten 135 sowie zur Decodierung der digitalen Produktkennzeichnung 130 der Intraokularlinse, insbesondere des Typs und der Brechkraft, zur Identifikation des ophthalmologischen Implantats 100 aus diesem Bild sowie eine Anzeige- und/oder Ausgabevorrichtung 240 zur Anzeige und/oder Ausgabe der decodierten Identifikationsdaten des ophthalmologischen Implantats 100 auf.

Dieses Ausführungsbeispiel des erfindungsgemäßen Maschinenlesesystems 200 kann dabei auch Intraokularlinsen decodieren, die bereits in ein Patientenauge 300 implantiert sind.

Die Fig. 11 zeigt die Nutzung eines digitalen Produktkennzeichnung 130, hier eines codierten Markers, insbesondere in Form des maschinenlesbaren codierten Punktrasters aus Markierungspunkten 135, zu einer Verifizierung des ophthalmologischen Implantats 100 während der Implantation und in-vivo nach der Implantation. Bereits während der Implantation und in-vivo nach der Implantation gibt es viele verschiedene Anlässe, bei denen eine Verifizierung des Implantats 100 und damit eine Rückverfolgbarkeit hilfreich ist. Dazu ist das ophthalmologische Implantat 100 über die digitale Produktkennzeichnung 130 verbindungsfähig zu verschiedenen Geräten wie dem Operationsmikroskop 250 und verschiedenen Diagnosegeräten 260. Diese nehmen kontaktlos Verbindung auf zur Registrierung 270, die sich auf einem internen Server oder aber in der Cloud befinden kann.

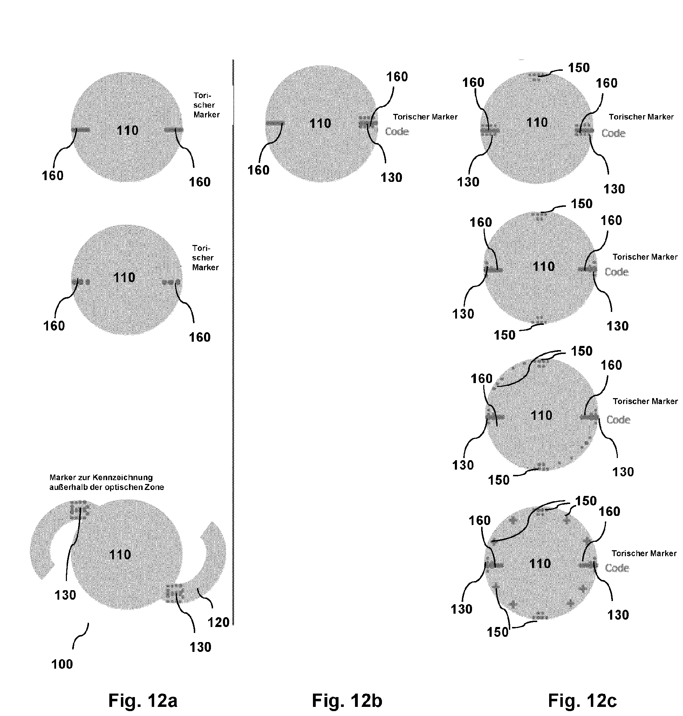

In der Fig 12a ist eine digitale Produktkennzeichnung 130 mit torischem Marker 160 auf einem ophthalmologischen Implantat 100 nach dem Stand der Technik dargestellt: Die digitale Produktkennzeichnung 130 befindet sich im den optisch abbildenden Element 110 nahen Bereich der Haptik 120 des ophthalmologischen Implantats 100. Torische Marker 160 sind am Rand des optisch abbildenden Elements 110 angeordnet

Die Fig. 12b zeigt eine digitale Produktkennzeichnung 130 mit torischem Marker 160 nach einem Ausführungsbeispiel des erfindungsgemäßen ophthalmischen Implantats 100, während in der Fig. 12c digitale Produktkennzeichnung 130 mit torischem Marker 160 und Ausrichtungshilfe 150 nach weiteren Ausführungsbeispielen des erfindungsgemäßen ophthalmischen Implantats 100 dargestellt sind.

Im Allgemeinen kann ein kodierter Marker auf einem ophthalmologischen Implantat 100, insbesondere auf einer IOL, jede Art von visuell erkennbarer kodierter Information sein, die die oben beschriebenen Funktionen bietet. Beispiele für solche kodierten Informationen sind u. a. (i) Standardcodes wie lineare Strichcodes oder Matrix-(2D)-Strichcodes einschließlich Punktcode, QR-Code oder (ii) fortgeschrittene Codes wie 3D-Matrixcodes. Die Codes können sich in der Anzahl, Größe oder Breite der (einzelnen) Elemente (z. B. Pixel), der Gesamtgröße des Codes, den Abständen zwischen den Elementen und der Ausrichtung der Elemente innerhalb des Codes unterscheiden. Der codierte Marker auf einer IOL wird durch ein maschinenlesbares Muster gebildet. Ein solches Muster kann unter verschiedenen Beleuchtungsarten erkannt werden, z. B. unter normaler Weißlichtbeleuchtung, Fluoreszenzbeleuchtung oder Laserbeleuchtung.

Der digitale Produktkennzeichnung 130 mittels codierte Marker ist nun so auf der IOL angebracht, dass sie während der Implantation und nach der Operation erkennbar ist. In den hier gezeigten Ausgestaltungen befindet sich der kodierte Marker am Rand des optischen abbildenden Elements 110 einer IOL, der in der Regel durch Pupillenerweiterung zugänglich ist. Der codierte Marker, insbesondere das maschinenlesbare codierte Punktraster aus Markierungspunkten 135 enthält Informationen wie die Spezifikationsdaten des jeweiligen ophthalmologischen Implantats 100 (im Falle einer IOL z. B. Dioptrie, Typ, Hersteller, Modell, Material, torische Achse). Die Spezifikationsdaten können aber auch durch eine eindeutige Kennung dargestellt werden, die den Abruf der Daten aus einer Datenbank ermöglicht.

Ein codierter Marker, wie er in der Erfindung vorgeschlagen wird, ermöglicht nicht nur eine zuverlässige Identifizierung der IOL, sondern in beispielhaften Ausführungsformen, wie in den Fig. 12b und 12c dargestellt, auch die Erkennung der IOL-Position während der Operation. Die Verwendung eines codierten Markers ermöglicht die Bereitstellung von IOL-Design-Spezifikationsdaten (einschließlich IOL-Geometrie) und tatsächlichen IOL-Positionsdaten. Die vom codierten Marker gelieferten Informationen eröffnen neue Möglichkeiten für die computergestützte Optimierung der IOL-Positionierung. Der codierte Marker stellt geometrische Daten des einzelnen Implantats 100 dar, die eine computergestützte Erkennung seiner Position ermöglichen. Aufgrund des codierten Charakters des Markers liefert selbst eine Untergruppe der erkannten Merkmale eines codierten Markers nützliche Informationen, um eine IOL stabiler und präziser im Auge auszurichten. Der codierte Marker umfasst Mittel zur Fehlererkennung, Fehlertoleranz und idealerweise zur Fehlerkorrektur.

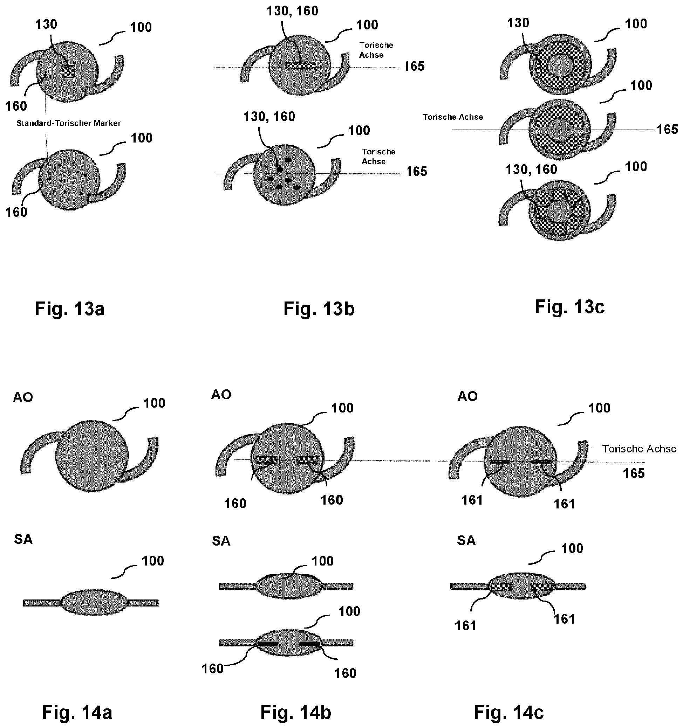

Die Fig. 13a bis 13c zeigen digitale Produktkennzeichnungen 130 mit torischem Marker 160 in der optisch wirksamen Zone des optisch abbildenden Elements 110 in weiteren verschiedenen Ausführungsbeispielen des erfindungsgemäßen ophthalmischen Implantats 100. Diese Figuren beschreiben Ausführungsformen von maschinenlesbaren codierten Punktrastern 135, also Data-Matrix-Codes bzw. codierte Marker, und deren Variationen für die Verwendung innerhalb der optisch wirksamen Zone 115 bzw. der optischen Zone von 4,4 mm. Dies ist von Vorteil, um im implantierten Zustand einen leichteren Zugang zum Code zu haben, da keine Pupillenerweiterung erforderlich ist. Dabei muss sichergestellt werden, dass keine negativen Auswirkungen auf die optische Leistung des ophthalmologischen Implantats 100, insbesondere einer Intraokularlinse, an den Patienten weitergegeben werden. Hier besteht die Lösung darin, den Abstand zwischen den Markierungspunkten zu vergrößern und/oder zu randomisieren und/oder die Größe der Markierungspunkte gering zu halten. In der Fig. 13a wird dabei ein im Stand der Technik üblicher torischer Marker 160 am äußersten Rand des optisch abbildenden Elements 110 in Kombination mit der hier erfindungsgemäß beschriebenen digitalen Produktkennzeichnung 130 in Form eines maschinenlesbaren codierten Punktrasters von Markierungspunkten 135 in der optisch wirksamen Zone 115 des optisch abbildenden Elements 110 des ophthalmologischen Implantats 100 dargestellt, während in der Fig. 13b der torische Marker 160 erfindungsgemäß in die digitalen Produktkennzeichnung 130 in Form eines maschinenlesbaren codierten Punktrasters von Markierungspunkten 135 in der optisch wirksamen Zone 115 des optisch abbildenden Elements 110 des ophthalmologischen Implantats 100 integriert ist. Insbesondere im unteren der beiden Beispiele findet die Ausrichtung nicht etwa nur an einer Makrostruktur (der Form des codierten Markers) statt, sondern die Markierungspunkte weisen eine elliptische Form auf, wobei die Langachse der Ellipse hier parallel der torischen Achse verläuft.

Um die Torizität einer Intraokularlinse oder andere Linsenmerkmale (wie Haptik oder Verbindungsstellen modularer Linsen) anzuzeigen, können die Punkte in diese Richtung verlängert werden. Darüber hinaus kann, wie in Fig. 13c gezeigt, ein optischer Dens-Code verwendet werden, um das Licht in einer gewünschten Weise zu blockieren, wie dies bei Lochblenden-IOLs zur Erzielung einer größeren Tiefenschärfe der Fall ist. Hier bilden die maschinenlesbaren codierten Punktraster aus Markierungspunkten 135 bzw. ganz allgemein die Data-Matrix-Codes die optische Maske, oder ein Code wird auf die lichtblockierende Maske einer Pinhole-IOL aufgebracht. Dies ist vorteilhaft, da die optischen Nachteile eines codierten Markers bzw. Data-Matrix-Codes auf einer Maske entfallen.

In den Fig. 14a bis 14c sind digitale Produktkennzeichnungen 130 mit torischem Marker in weiteren verschiedenen Ausführungsbeispielen des erfindungsgemäßen ophthalmischen Implantats - für die Nutzung aus der Ansicht von oben AO sowie für die Nutzung in axialer Ansicht SA - dargestellt.

Der torische Marker 160, 161 befindet sich auf der Oberfläche des optisch abbildenden Elements 110 des ophthalmologischen Implantats 100, insbesondere einer Intraokularlinse, oder im Volumen des optisch abbildenden Elements 110 (d.h. im Material). Der torische Marker 160, hier beispielsweise in Form eines QR-Codes, ist vorzugsweise in der Ansicht von oben, AO lesbar, wie in der Fig. 14b gezeigt, was den Zugang über eine Kamera in der Produktion, in der Logistik und im implantierten Zustand über ein Operationsmikroskop 250, eine Spaltlampe 260 oder ein Biometriegerät 260 ermöglicht. Alternativ ist der torische Marker 161, ggf auch der Data-Matrix-Code, in einer axialen Ansicht (also einer Seitenansicht, SA) lesbar, wie in der Fig. 14c gezeigt, die bei tomographischen Messungen zugänglich ist. In dieser Ausführungsform ist der Code in der Draufsicht (Ansicht von oben, AO) nicht lesbar, hat aber weniger Einfluss auf die Dysphotopsie im implantierten Zustand.

Im Allgemeinen sind torische Marker 160 für torische IOLs weithin etabliert, ohne dass über Dysphotopsie berichtet wird, was den Vorteil hat, dass diese Form und Fläche für einen codierten Marker in Form eines Data-Matrix-Code im implantierten Zustand einer IOL mit erweiterten Pupillen verwendet werden kann. Diese Erfindung kann für alle IOLs, nicht nur für torische IOLs, verwendet werden und ermöglicht es, die Rotation des ophthalmologischen Implantats 100 zu verfolgen.

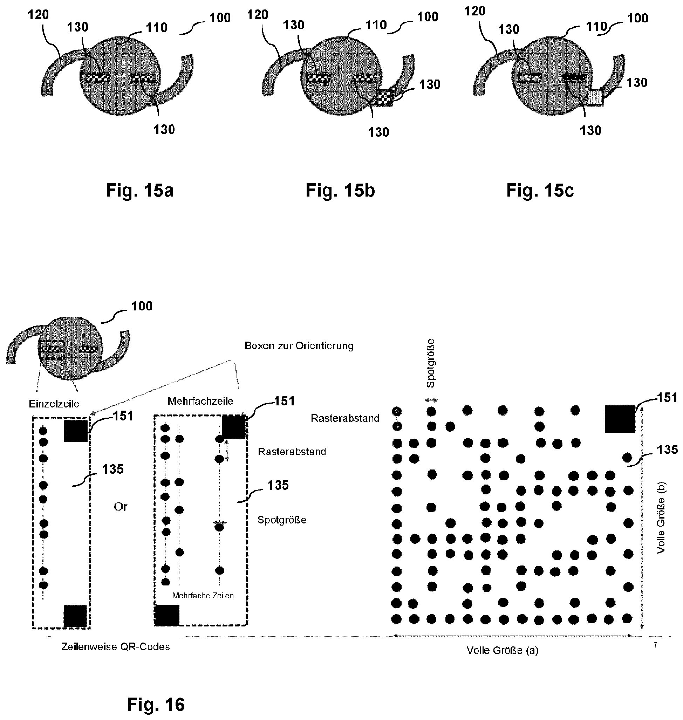

In den Fig. 15a bis 15c ist eine digitale Produktkennzeichnung 130 in Form eines codierten Markers, insbesondere eines maschinenlesbaren codierten Punktrasters aus Markierungspunkten 135 in weiteren verschiedenen Ausführungsbeispielen des erfindungsgemäßen ophthalmischen Implantats 100 dargestellt.

Die Fig. 15a zeigt dabei zwei identische längliche codierte Marker in Form von QR-Codes auf einer Intraokularlinse 100. Da ein klassischer torischer Marker 160 zwei Linien enthält, können beide als UDI verwendet werden. Zusätzliche Codes auf der Fläche der Haptik 120 können von Vorteil sein. Alle Codes können den gleichen Inhalt oder unterschiedliche Inhalte haben, um die Datendichte eines Codes zu verringern. In der Fig. 15b sind zwei identische längliche codierte Marker in Form von QR-Codes auf einer Intraokularlinse 100 in Kombination mit einem QR-code auf der Haptik gezeigt. In der Fig. 15c sind zwei nicht-identische längliche codierte Marker in Form von QR-Codes auf einer Intraokularlinse 100 in Kombination mit einem QR-code auf der Haptik dargestellt.

Die Fig. 16 zeigt verschiedene Anordnungen des maschinenlesbaren codierten Punktrasters aus Markierungspunkten 135 einer digitalen Produktkennzeichnung 130. In diesem Fall handelt es sich um QR-Code-Designs von rechteckiger oder quadratischen Form in UDI-konformem Rastermuster, das einfacher erzeugbar ist als das der Fig. 6 und 7, aber mit dem eine gewisse Periodizität erhalten bleibt. Die Anzahl der Zeilen der Daten-Matrix-Codes variiert hier zwischen den einzelnen Beispielen. Zusätzliche Merkmale zeigen die Ausrichtung der Data-Matrix-Codes, was die Erkennung von Geräten verbessert, die zum Drehen und Ausrichten der IOL während der Operation verwendet werden. Hier können zusätzliche Funktionen von Mikroskopen zum Ausrichten der IOL verwendet werden. Die hier gezeigten Codes weisen - neben der Tatsache, dass eine prinzipielle Ausrichtung anhand des maschinenlesbaren codierten Punktrasters aus Markierungspunkten 135 prinzipiell möglich ist - zusätzliche Orientierungsboxen 151 auf, die die Ausrichtung des entsprechenden ophthalmologischen Implantats 100 unterstützen.

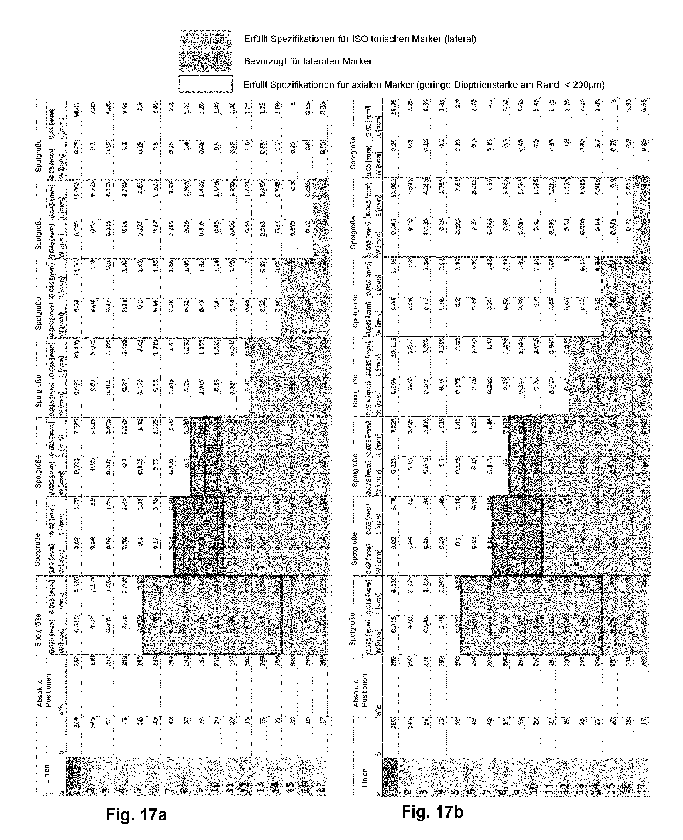

In den Tabellen der Fig. 17a und 17b ist eine Größenabschätzung für M3 bzw. M4 UDI Codes dargestellt. Um die Größe eines codierten Markers, aber beispielsweise auch eines torischen Markers abzuschätzen, werden Berechnungen für verschiedene Markierungspunktgrößen, die Anzahl der Spalten und die Anzahl der Zeilen durchgeführt. Für einen M3-Code (siehe Fig. 17a) und einen M4-Code (siehe Fig 17b, wobei M4 den vollständigen Inhalt der UDI, einschließlich SN, Herstellungsdatum, Herstellungsort und Ablaufdatum sowie eine Prüfsummenlänge umfasst) werden verschiedene Kombinationen von Zeilen-, Spalten- und Punktgrößen für die Draufsicht AO und die axiale Ansicht SA gezeigt. Es gibt Kombinationen von Zeilen, Spalten und Punktgrößen, die die Vorgaben der ISO-Norm erfüllen, um einen entsprechenden Marker auf dem optisch abbildenden Element 110, insbesondere in dessen Randbereich, anzuordnen. Eine kleine Spotgröße von 20µm/25µm bis 50µm ist jedoch auch in Bezug auf Auflösung und Kontrast von Vorteil. Die bevorzugten Kombinationen sind den Fig. 17a und 17b hervorgehoben. Für die konventionelle OCT ist eine axiale Auflösung von ~5µm möglich. Dies wäre eine untere Grenze für die Punktgrößen.

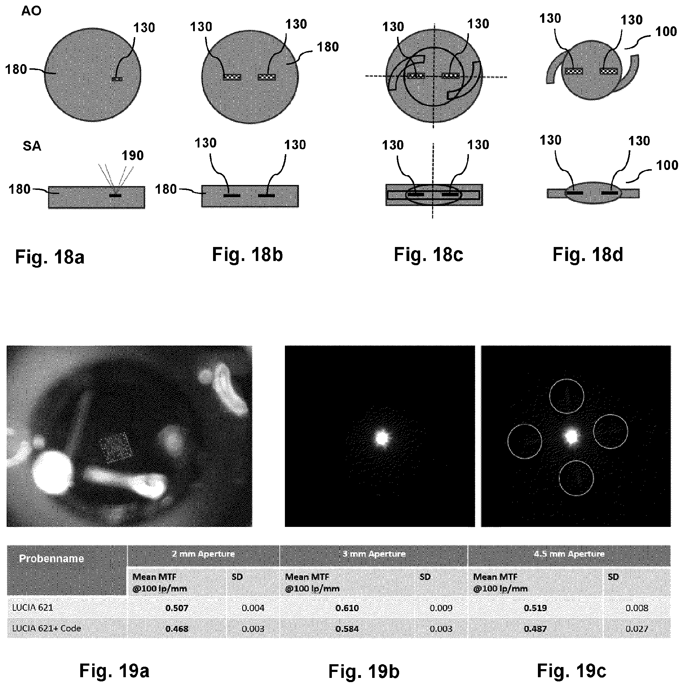

Zur Rückverfolgbarkeit eines ophthalmologischen Implantats 100 bis zur Herstellung, insbesondere des optisch abbildenden Elements 110 in der Fertigung können codierte Marker, insbesondere ein maschinenlesbares codiertes Punktraster von Markierungspunkten 135 als digitale Produktkennzeichnung 130 durch Lasergravur mit einem Laser 190 in oder auf das Material aufgebracht werden. Die Fig. 18a bis 18d beschreiben eine bevorzugte Variante eines Herstellungsverfahrens einer Intraokularlinse mit einem codierten Marker als digitale Produktkennzeichnung 130, aber auch als Ausrichtungshilfe bzw. als torischer Marker, der im Volumen des optisch abbildenden Elements 110 angeordnet ist. Die Codes werden hier zunächst in den Materialrohling 180 (auch Blank genannt) geschrieben, siehe Fig. 18a und 18b. Da sich der Code bzw. die Codes im Inneren des Materials befinden, kann das ophthalmologische Implantat 100 schon während des gesamten Herstellungsprozesses, einschließlich Drehen, Fräsen, Sterilisieren und Verpacken, nachverfolgt werden, siehe Fig. 18c und 18d. Im Material ist der Code vor jeglichem Abrieb während des Diamantdrehens und -fräsens geschützt. Der Code der digitalen Produktkennzeichnung 130 befindet sich in dem hier gezeigten Beispiel an der Stelle, an der sich die torischen Marker 160 im fertigen Produkt befinden werden.

Ein weiterer Vorteil ist, dass ein Data-Matrix-Code im Inneren des Materials das Auslesen bei der Herstellung, der Logistik und im implantierten Zustand verbessert. Beispielsweise können torische Marker 160 auf der Oberfläche des optisch abbildenden Elements 110, insbesondere auf einer Linsenoberfläche, bei hohen Dioptrien und der Hornhaut aufgrund der optischen Projektion deformiert aussehen, was die Ausrichtung bei der Implantation des ophthalmologischen Implantats 100 erschwert. Die torischen Marker 160 können zudem zur Ausrichtung des Materialrohlings 180 bei der Herstellung des ophthalmologischen Implantats 100 verwendet werden, um sicherzustellen, dass Torizität und Haptik in der richtigen Achse liegen.

Es sollte erwähnt werden, dass der Bearbeitungsvorgang bezüglich Kippung und Zentrierung, insbesondere aber für die taktgenaue Ausrichtung einer nicht sphärischen Intraokularlinse sehr präzise sein muss. Für den Fall, dass die Linse nicht perfekt ausgerichtet ist, muss diese Information mit der Seriennummer verknüpft und sowohl online (Zugriff über die Datenbank) als auch offline (Kartonetikett) gespeichert werden.

Alternativ kann der Marker auch direkt nach der Bearbeitung oder nach dem Polieren des ophthalmologischen Implantats 100 angebracht werden. Hierbei kann der Marker immer noch in das Material eingebettet oder auf die Oberfläche aufgebracht werden.

Neben der klassischen Lasergravur können auch alternative Techniken zur Herstellung der gezeigten Daten-Matrix-Codes verwendet werden. Eine Möglichkeit wäre die photochemische Erzeugung von Metall- (z. B. Silber, Gold) oder Silikon-Nanopartikeln innerhalb der Biomaterialmatrix durch Zwei-Photonen-Absorption.

Die Fig. 19a bis 19c zeigen Aufnahmen einer in ein ISO-Auge implantierten Intraokularlinse Zeiss Lucia 621 mit einem codierten Marker auf der Oberfläche des optisch abbildenden Elements 110 (Fig. 19a) und den Halo/Glare Test ohne diesen codierten Marker (Fig. 19b) wie auch mit diesem codierten Marker (Fig. 19c). Hierbei handelt es sich um einen lasergravierten QR-Code (M4) auf der IOL-Oberfläche in einem ISO-Auge, das für Prüfstandsversuche verwendet wurde. Der Code wurde als 50-µm-Standardmuster zur eindeutigen Gerätekennung (UDI) direkt in der Mitte der optisch wirksamen Zone 115 eingebracht, getestet und analysiert.

Der Code ist durch ein Mikroskop gut sichtbar. Die Werte der Modulationstransferfunktion (MTF) für 100 lp/mm liegen nahe an denen einer nicht mit einem codierten Marker versehenen IOL. Es sind also mit diesem klassischen lasergravierten Code nur geringe Probleme in Bezug auf Halo, Blendung und Visusminderung festzustellen. Aufgrund der Periodizität des Codes sind kleine Gitternetzeffekte (kleine Dysphotopsien) sichtbar. Die Testlimits für ein solches ophthalmologisches Implantat 100 werden jedoch bestanden. Diese Effekte könnten durch die Positionierung an der Peripherie des optisch abbildenden Elements 110 bzw. weiterer Randomisierung des Musters bei der Erstellung der digitalen Produktkennzeichnung 130 weiter minimiert werden.

Da die Markierungspunkte in einer Fourier-Ebene liegen, werden sie nicht scharf auf die Netzhaut projiziert. Hier wird nur die Sehschärfe durch die Art der "Blockierung" von Lichtstrahlen in der zentralen optischen Zone verringert, was zu einem Kontrastverlust führt. Bei einer Schätzung eines M4-Codes (17x17) mit einer quadratischen Markierungspunktgröße von 25 µm und einer zentralen optischen Zone von 4,4 mm werden etwa 0,5 % des Lichts blockiert, was vernachlässigbar ist und keinen Einfluss auf die MTF-Funktion hat. Um Beugungseffekte durch die Periodizität des Punktmusters des Codes zu reduzieren, muss der Gitterabstand größer sein als die Größe der Markierungspunkte (gelegentlich auch Spotgröße genannt). Dies ist im vorliegenden Beispiel der Fall.

### Bezugszeichen

- 0 bis 36:: Nummern der Basisgitterpunkte
- 1.1, 1.2, 1.3, 1.4:: mögliche Positionen des Markierungspunktes des ersten Basisgitterpunktes

- 51: Sektor / Zelle
- 52: radiale Zone
- 53: radiale Richtung
- 54: sektorale Richtung
- 55: Kreuzungspunkt / mögliche Position für Markierungspunkt
- 56: Basisgitterpunkt
- 57: Markierungspunkt
- 58: Referenzmarke

- 100: ophthalmologisches Implantat
- 110: optisch abbildendes Element
- 115: optisch wirksame Zone
- 120: Haptik
- 130: digitale Produktkennzeichnung
- 135: maschinenlesbares codiertes Punktraster aus Markierungspunkten
- 140: virtuelles polares oder kartesisches Basisgitter
- 150: Ausrichtungshilfe
- 151: Orientierungsbox
- 160: torischer Marker für Ansicht von oben
- 161: torischer Marker für Ansicht von der Seite / axiale Ansicht
- 165: torische Achse
- 180: Blank / Materialrohling
- 190: Laser

- 200: Maschinenlesesystem
- 210: Beleuchtungssystem
- 220: Kamerasystem
- 230: Analyseeinheit
- 240: Ausgabevorrichtung
- 250: Operationsmikroskop
- 260: Diagnosegerät
- 270: Registrierung

- 300: Patientenauge

- AO: Ansicht von oben / laterale Ansicht
- SA: Seitenansicht / axiale Ansicht

## Patentansprüche

1. Ophthalmologisches Implantat (100), insbesondere Intraokularlinse, umfassend ein optisch abbildendes Element (110), insbesondere eine zentrale optische Linse, das eine optisch wirksamen Zone (115) aufweist, und bevorzugt eine Haptik (120), die sich dem optisch abbildenden Element (110) anschließt, wobei auf dem optisch abbildenden Element (110) und/oder im optisch abbildenden Element (110) eine digitale Produktkennzeichnung (130) des ophthalmologischen Implantats (100), insbesondere des Typs und der Brechkraft und/oder ein Datenbankschlüssel, angeordnet ist,
**dadurch gekennzeichnet, dass** die digitale Produktkennzeichnung (130) innerhalb der optisch wirksamen Zone (115) des optisch abbildenden Elements mittels eines im Bereich des sichtbaren Lichts maschinenlesbaren codierten Punktrasters (135) aus Markierungspunkten (57) realisiert ist, das einen pseudozufälligen unregelmäßigen Charakter hat, wodurch eine Anzahl verschiedener definierter Abweichungen der Markierungspunkte von einem festen Bezugspunkt, der einen regelmäßigen Charakter des Punktrasters erzeugen würde, besteht.

2. Ophthalmologisches Implantat (100) nach Anspruch 1, dessen maschinenlesbares codiertes Punktraster (135) aus Markierungspunkten (57) zentral innerhalb der optisch wirksamen Zone (115) des optisch abbildenden Elements (110) angeordnet ist.

3. Ophthalmologisches Implantat (100) nach Anspruch 1 oder 2, wobei das codierte Punktraster (135) aus Markierungspunkten (57) so aufgebaut ist, dass ein virtuelles polares oder kartesisches Basisgitter (140) auf dem optischen abbildenden Element (110), bevorzugt auf der optischen Zone (115) des optisch abbildenden Elements (110), derart angeordnet ist, das dadurch gleichartige Sektoren (51) bzw. gleichartige Zellen (51') mit jeweils einem definierten Basisgitterpunkt (56) des Sektors (51) bzw. der Zelle (51') beschrieben werden, und ein realer Markierungspunkt (57) des Punktrasters (135) in jedem Sektor (51) bzw. jeder Zelle (51') in einer Position (55) angeordnet ist, die einen Versatz zu diesem Basisgitterpunkt (56) aufweist, wobei der Versatz in jedem Sektor (51) bzw. jeder Zelle (51') in einer von vier möglichen Richtungen, von denen vorzugsweise je zwei einander entgegengesetzt verlaufen, erfolgt, und einen definierten Abstand zum Basisgitterpunkt (56) aufweist.

4. Ophthalmologisches Implantat (100) nach Anspruch 3, wobei ein Sektor (51) bzw. eine Zelle (51') mit einem Basisgitterpunkt (56) vier Zustände zur Verfügung stellt, die durch die jeweilige Lage des Markierungspunktes (57) in einer von vier Positionen (55) um den Basisgitterpunkt (56) gekennzeichnet sind.

5. Ophthalmologisches Implantat (100) nach Anspruch 4, wobei ein Sektor (51) bzw. eine Zelle (51') mit einem Basisgitterpunkt (56) einen fünften Zustand zur Verfügung stellt, der durch das Nichtvorhandensein eines Markierungspunktes (57) an einer der vier möglichen Positionen (55) um einen Basisgitterpunkt (56) definiert ist.

6. Ophthalmologisches Implantat (100) nach Anspruch 4 oder 5, wobei in einem Sektor (51) bzw. in einer Zelle (51') mit einem Basisgitterpunkt (56) weitere Zustände definiert sind durch weitere Versatzrichtungen und/oder weitere definierte Abstände des Versatzes des Markierungspunktes (57) zum Basisgitterpunkt (56).

7. Ophthalmologisches Implantat (100) nach einem der Ansprüche 1 bis 6, wobei der Anteil der Fläche der Markierungspunkte (57) zur Gesamtfläche des optisch abbildenden Elements (110) kleiner 2%, insbesondere kleiner 1% und besonders bevorzugt kleiner 0,5% ist, und/oder wobei der Anteil der Fläche der Markierungspunkte (57) zur Fläche der optisch wirksamen Zone des optisch abbildenden Elements (110) kleiner 8%, insbesondere kleiner 4% und besonders bevorzugt kleiner 2% ist.

8. Ophthalmologisches Implantat (100) nach einem der Ansprüche 1 bis 7, wobei das maschinenlesbare codierte Punktraster (135) strukturelle Markierungspunkte (57) aufweist.

9. Ophthalmologisches Implantat (100) nach einem der Ansprüche 1 bis 8, das eine ergänzte Produktkennzeichnung (130') enthält, die neben der ursprünglichen Produktkennzeichnung (130) Informationen für Prüfsummen und Fehlerkorrekturverfahren aufweist.

10. Ophthalmologisches Implantat (100) nach einem der Ansprüche 1 bis 9, das in einem definierten Abstand zum maschinenlesbaren codierten Punktraster (135) aus Markierungspunkten (57) eine oder mehrere Referenzmarken (58) aufweist.

11. Ophthalmologisches Implantat (100) nach einem der Ansprüche 1 bis 10, das eine Ausrichtungshilfe (150) aufweist, wobei die Ausrichtungshilfe (150) ebenfalls vorzugsweise innerhalb der optisch wirksamen Zone (115) angeordnet und bevorzugt das maschinenlesbare codierte Punktraster (135) aus Markierungspunkten (57) enthält oder aus diesem besteht.

12. Ophthalmologisches Implantat (100) nach einem der Ansprüche 1 bis 11, der einen torischen Marker (160), der in der Ansicht von oben lesbar ist, und/oder einen torischen Marker (161), der in einer axialen Ansicht lesbar ist, aufweist.

13. Maschinenlesesystem (200) zur Erfassung und Decodierung der digitalen Produktkennzeichnung (130) in Form eines codierten Punktrasters (135) aus Markierungspunkten (57) auf einem ophthalmologischen Implantat (100) nach einem der Ansprüche 1 bis 12, umfassend
- ein Kamerasystem (220) zur Aufnahme von Strukturen des maschinenlesbaren codierten Punktrasters (135) aus Markierungspunkten (57) auf dem ophthalmologischen Implantat (100), und
- eine Analyseeinheit (230) zur Erfassung und Auswertung eines vom Kamerasystem (220) aufgenommenen Bildes der Strukturen des maschinenlesbaren codierten Punktrasters (135) aus Markierungspunkten (57) sowie zur Decodierung der digitalen Produktkennzeichnung (130) des ophthalmologischen Implantats (100).

14. Maschinenlesesystem (200) nach Anspruch 13, dass mit einem Datenbanksystem verbunden ist, wobei die digitale Produktkennzeichnung (130) einen Datenbankschlüssel enthält und dem Datenbankschlüssel im Datenbanksystem die Produktinformationen des ophthalmologischen Implantats zugeordnet sind.

15. Maschinenlesesystem (200) nach Anspruch 13 oder 14, das Teil eines Operationsmikroskops (250) oder einer Spaltlampe ist.

## Claims

1. Ophthalmological implant (100), in particular an intraocular lens, comprising an optically imaging element (110), in particular a central optical lens having an optically effective zone (115), and preferably comprising a haptic (120) adjoining the optically imaging element (110), with a digital product identification (130) of the ophthalmological implant (100), in particular the type and the refractive power and/or a database key, being arranged on the optically imaging element (110) and/or in the optically imaging element (110),
**characterized in that** the digital product identification (130) is realized within the optically effective zone (115) of the optically imaging element by means of a coded point grid (135) made of marking points (57) that is machine-readable in the visible light range and has a pseudo-random, irregular character, whereby there is a number of different defined deviations of the marking points from a fixed reference point, which would produce a regular character of the point grid.

2. Ophthalmological implant (100) according to Claim 1, whose machine-readable coded point grid (135) made of marking points (57) is arranged centrally within the optically effective zone (115) of the optically imaging element (110).

3. Ophthalmological implant (100) according to Claim 1 or 2, wherein the coded point grid (135) is constructed from marking points (57) such that
a virtual polar or Cartesian base lattice (140) is arranged on the optically imaging element (110), preferably on the optical zone (115) of the optically imaging element (110), in such a way that this describes similar sectors (51) or similar cells (51'), each with a defined base lattice point (56) of the sector (51) or the cell (51'), and a real marking point (57) of the point grid (135) is arranged in each sector (51) or each cell (51') at a position (55) which has an offset to this base lattice point (56), the offset in each sector (51) or each cell (51') being in one of four possible directions, which preferably run pairwise opposite to each other, and having a defined distance to the base lattice point (56).

4. Ophthalmological implant (100) according to Claim 3, wherein a sector (51) or cell (51') with a base lattice point (56) provides four states which are **characterized by** the respective location of the marking point (57) in one of four positions (55) around the base lattice point (56).

5. Ophthalmological implant (100) according to Claim 4, wherein a sector (51) or cell (51') with a base lattice point (56) provides a fifth state defined by the absence of a marking point (57) at one of the four possible positions (55) around a base lattice point (56).

6. Ophthalmological implant (100) according to Claim 4 or 5, wherein further states are defined in a sector (51) or cell (51') with a base lattice point (56) by way of further offset directions and/or further defined distances of the offset of the marking point (57) to the base lattice point (56).

7. Ophthalmological implant (100) according to any of Claims 1 to 6, wherein the proportion of the area of the marking points (57) to the total area of the optically imaging element (110) is less than 2%, in particular less than 1%, and particularly preferably less than 0.5%, and/or wherein the proportion of the area of the marking points (57) to the area of the optically effective zone of the optically imaging element (110) is less than 8%, in particular less than 4%, and particularly preferably less than 2%.

8. Ophthalmological implant (100) according to any of Claims 1 to 7, wherein the machine-readable coded point grid (135) has structural marking points (57).

9. Ophthalmological implant (100) according to any of Claims 1 to 8, containing a supplemented product identification (130') which, in addition to the original product identification (130), has information for checksums and error correction methods.

10. Ophthalmological implant (100) according to any of Claims 1 to 9, having one or more reference marks (58) at a defined distance from the machine-readable coded point grid (135) made of marking points (57).

11. Ophthalmological implant (100) according to any of Claims 1 to 10, having an alignment aid (150), the alignment aid (150) likewise preferably being arranged within the optically effective zone (115) and preferably containing or consisting of the machine-readable coded point grid (135) made of marking points (57).

12. Ophthalmological implant (100) according to any of Claims 1 to 11, having a toric marker (160) which is readable in the view from above and/or a toric marker (161) which is readable in an axial view.

13. Machine reading system (200) for capturing and decoding the digital product identification (130) in the form of a coded point grid (135) made of marking points (57) on an ophthalmological implant (100) according to any of Claims 1 to 12, comprising
- a camera system (220) for recording structures of the machine-readable coded point grid (135) made of marking points (57) on the ophthalmological implant (100), and
- an analysis unit (230) for capturing and evaluating a camera system (220) recorded image of the structures of the machine-readable coded point grid (135) made of marking points (57), and for decoding the digital product identification (130) of the ophthalmological implant (100).

14. Machine reading system (200) according to Claim 13, connected to a database system, wherein the digital product identification (130) contains a database key and the database key is assigned to the product information of the ophthalmological implant in the database system.

15. Machine reading system (200) according to Claim 13 or 14, which is part of a surgical microscope (250) or a slit lamp.

## Revendications

1. Implant ophtalmologique (100), en particulier lentille intraoculaire, comprenant un élément d'imagerie optique (110), en particulier une lentille optique centrale, qui comporte une zone optiquement active (115), et de manière préférée un élément haptique (120), qui se raccorde à l'élément d'imagerie optique (110), une identification de produit numérique (130) de l'implant ophtalmologique (100), en particulier du type et de la force réfractive et/ou une clé de base de données étant disposées sur l'élément d'imagerie optique (110) et/ou dans l'élément d'imagerie optique (110),
**caractérisé en ce que** l'identification de produit numérique (130) est réalisée à l'intérieur de la zone optiquement active (115) de l'élément d'imagerie optique au moyen d'une grille de points (135) codée, composée de points de marquage (57), lisible par une machine dans la plage de la lumière visible, , laquelle a un caractère irrégulier pseudo-aléatoire, créant ainsi un certain nombre d'écarts définis différents des points de marquage par rapport à un point de référence fixe, qui produirait un caractère régulier de la grille de points.

2. Implant ophtalmologique (100) selon la revendication 1, dont la grille de points (135) codée, composée de points de marquage (57), lisible par une machine, est disposée au centre à l'intérieur de la zone optiquement active (115) de l'élément d'imagerie optique (110).

3. Implant ophtalmologique (100) selon la revendication 1 ou 2, dont la grille de points (135) codée, composée de points de marquage (57), est élaborée de telle manière que
une grille de base (140) virtuelle polaire ou cartésienne est disposée sur l'élément d'imagerie optique (110), de manière préférée sur la zone optique (115) de l'élément d'imagerie optique (110) de telle manière que des secteurs ainsi similaires (51) ou des cellules similaires (51') sont décrits avec un point de grille de base (56), défini du secteur (51) ou de la cellule (51'), et un point de marquage (57) réel de la grille de points (135) est disposé dans chaque secteur (51) ou chaque cellule (51') dans une position (55), qui présente un décalage par rapport audit point de grille de base (56), le décalage dans chaque secteur (51) ou chaque cellule (51') étant réalisé dans une des quatre directions possibles, dont deux de préférence s'étendent à l'opposé l'une de l'autre, et présente une distance définie par rapport au point de grille de base (56).

4. Implant ophtalmologique (100) selon la revendication 3, **caractérisé en ce qu'**un secteur (51) ou une cellule (51') avec un point de grille de base (56) fournit quatre états, qui sont **caractérisés par** la position respective du point de marquage (57) dans une des quatre positions (55) autour du point de grille de base (56).

5. Implant ophtalmologique (100) selon la revendication 4, **caractérisé en ce qu'**un secteur (51) ou une cellule (51') avec un point de grille de base (56) fournit un cinquième état, qui est défini par l'absence d'un point de marquage (57) sur une des quatre positions (55) possibles autour d'un point de grille de base (56).

6. Implant ophtalmologique (100) selon la revendication 4 ou 5, **caractérisé en ce que** d'autres états sont définis dans un secteur (51) ou dans une cellule (51') avec un point de grille de base (56) par d'autres directions de décalage et/ou d'autres distances définies du décalage du point de marquage (57) par rapport au point de grille de base (56).

7. Implant ophtalmologique (100) selon l'une des revendications 1 à 6, **caractérise en ce que** la proportion de la surface des points de marquage (57) par rapport à la surface totale de l'élément d'imagerie optique (110) est inférieure à 2 %, en particulier inférieure à 1 % et de manière particulièrement préférée inférieure à 0,5 %, et/ou que la proportion de la surface des points de marquage (57) par rapport à la surface de la zone optiquement active de l'élément d'imagerie optique (110) est inférieure à 8 %, en particulier inférieure à 4 % et de manière particulièrement préférée inférieure à 2 %.

8. Implant ophtalmologique (100) selon l'une des revendications 1 à 7, dont la grille de points (135) codée est lisible par une machine, comporte des points de marquage (57) structurels.

9. Implant ophtalmologique (100) selon l'une des revendications 1 à 8, qui contient une identification de produit supplémentaire (130') qui, en plus de l'identification de produit d'origine (130), comporte des informations pour des sommes de contrôle et des procédés de correction d'erreurs.

10. Implant ophtalmologique (100) selon l'une des revendications 1 à 9, qui comporte un ou plusieurs repères de référence (58) à une distance définie de la grille de points (135) codée, composée de points de marquage (57), lisible par une machine.

11. Implant ophtalmologique (100) selon l'une des revendications 1 à 10, qui comporte une aide à l'alignement (150), l'aide à l'alignement (150) également étant disposée de préférence à l'intérieur de la zone optiquement active (115) et contenant de manière préférée la grille de points (135) codée, composée de points de marquage (57), lisible par une machine ou en étant constituée.

12. Implant ophtalmologique (100) selon l'une des revendications 1 à 11, qui comporte un marqueur torique (160), qui est lisible dans la vue de dessus, et/ou un marqueur torique (161), qui est lisible dans une vue axiale.

13. Système de lecture par une machine (200) destiné à détecter et décoder l'identification de produit numérique (130) sous la forme d'une grille de points (135) codée, composée de points de marquage (57), sur un implant ophtalmologique (100) selon l'une des revendications 1 à 12, comprenant
- un système de caméra (220) pour enregistrer des structures de la grille de points (135) codée de points de marquage (57), lisible par une machine composée, sur l'implant ophtalmologique (100), et
- une unité d'analyse (230) pour détecter et évaluer une image des structures de la grille de points (135) codée composée de points de marquage (57), enregistrée par le système de caméra (220), lisible par une machine, ainsi que pour décoder l'identification de produit numérique (130) de l'implant ophtalmologique (100).

14. Système de lecture par une machine (200) selon la revendication 13, qui est reliée à un système de base de données, l'identification de produit numérique (130) contenant une clé de base de données et les informations de produit de l'implant ophtalmologique étant associées à la clé de base de données dans le système de base de données.

15. Système de lecture par une machine (200) selon la revendication 13 ou 14, qui fait partie d'un microscope opératoire (250) ou d'une lampe à fente.
